# EUROPÄISCHE PATENTSCHRIFT

(11) **EP 1 546 394 B1**
(45) Veröffentlichungstag und Bekanntmachung des Hinweises auf die Patenterteilung: **06.12.2006**
(21) Anmeldenummer: 03769338.9
(22) Anmeldetag: 26.09.2003
(51) Int. Cl.: C12Q 1/68

(54) **VERFAHREN ZUM NACHWEIS VON SNPS AUF POLYDIMENSIONALEN MICROARRAYS**
METHOD FOR DETECTING SINGLE NUCLEOTIDE POLYMORPHISMS ON POLYDIMENSIONAL MICROARRAYS
PROCEDE D'IDENTIFICATION DE POLYMORPHISMES NUCLEOTIDIQUES SIMPLES (SNPS) SUR DES MICRORESEAUX POLYDIMENSIONNELS

(30) Priorität: 27.09.2002 DE 10245145
(43) Veröffentlichungstag der Anmeldung: 29.06.2005
(73) Patentinhaber: Array-on GmbH, 06466 Gatersleben (DE)
(72) Erfinder: FISCHER, Dirk, 06466 Gatersleben (DE); GEISTLINGER, Jörg, 06466 Gatersleben (DE)
(74) Vertreter: Neuefeind, Regina
(86) Internationale Anmeldenummer: PCT/EP2003/010773
(87) Internationale Veröffentlichungsnummer: WO 2004/031406

(56) Entgegenhaltungen:
- WO-A-00/58516
- WO-A-91/13075
- WO-A-95/00669
- WO-A-98/59066
- US-A- 5 759 777
- US-B1- 6 268 147
- CAI H. ET AL.: "Flow Cytometry-Based Minisequencing: A New Platform for High-Throughput Single-Nucleotide Polymorphism Scoring" GENOMICS, ACADEMIC PRESS, SAN DIEGO, US, Bd. 66, Nr. 2, 1. Juni 2000 (2000-06-01), Seiten 135-143, XP004439348 ISSN: 0888-7543
- HIRSCHHORN J.N. ET AL.: "SBE-TAGS: An array-based method for efficient single-nucleotide polymorphism genotyping" PROCEEDINGS OF THE NATIONAL ACADEMY OF SCIENCES OF USA, WASHINGTON, US, Bd. 97, Nr. 22, 24. Oktober 2000 (2000-10-24), Seiten 12164-12169, XP002158215 ISSN: 0027-8424
- SCHULZE A. ET AL.: "Navigating gene expression using microarrays--a technology review" NATURE CELL BIOLOGY, MACMILLAN PUBLISHERS, GB, Bd. 3, Nr. 8, August 2001 (2001-08), Seiten E190-E195, XP002259315 ISSN: 1465-7392
- JALANKO A. ET AL.: "SCREENING FOR DEFINED CYSTIC FIBROSIS MUTATIONS BY SOLID-PHASE MINISEQUENCING" CLINICAL CHEMISTRY, AMERICAN ASSOCIATION FOR CLINICAL CHEMISTRY. WINSTON, US, Bd. 38, Nr. 1, 1992, Seiten 39-43, XP001105344 ISSN: 0009-9147
- SYVANEN A.-C.: "FROM GELS TO CHIPS: MINISEQUENCING PRIMER EXTENSION FOR ANALYSIS OF POINT MUTATIONS AND SINGLE NUCLEOTIDE POLYMORPHISMS" HUMAN MUTATION, WILEY-LISS, NEW YORK, US, Bd. 13, Nr. 1, 1999, Seiten 1-10, XP000953256 ISSN: 1059-7794
- CHEN X. AND KWOK P.-Y.: "TEMPLATE-DIRECTED DYE-TERMINATOR INCORPORATION (TDI) ASSAY: A HOMOGENEOUS DNA DIAGNOSTIC METHOD BASED ON FLUORESCENCE RESONANCE ENERGY TRANSFER" NUCLEIC ACIDS RESEARCH, Bd. 25, Nr. 2, Januar 1997 (1997-01), Seiten 347-353, XP002125080 ISSN: 0305-1048
- PASTINEN T. ET AL.: "A SYSTEM FOR SPECIFIC, HIGH-THROUGPUT GENOTYPING BY ALLELE-SPECIFIC PRIMER EXTENSION ON MICROARRAYS" GENOME RESEARCH, Bd. 10, Nr. 7, Juli 2000 (2000-07), Seiten 1031-1042, XP008013561 ISSN: 1088-9051
- SHUMAKER J.M. ET AL.: "MUTATION DETECTION BY SOLID PHASE PRIMER EXTENSION" HUMAN MUTATION, WILEY-LISS, NEW YORK, US, Bd. 7, 1996, Seiten 346-354, XP001073481 ISSN: 1059-7794
- SYVANEN A.-C. ET AL.: "IDENTIFICATION OF INDIVIDUALS BY ANALYSIS OF BIALLELIC DNA MARKERS,USING PCR AND SOLID-PHASE MINISEQUENCING" AMERICAN JOURNAL OF HUMAN GENETICS, UNIVERSITY OF CHICAGO PRESS, CHICAGO,, US, Bd. 52, Nr. 1, 1993, Seiten 46-59, XP002050638 ISSN: 0002-9297
- PASTINEN T. ET AL.: "Multiplex, fluorescent, solid-phase minisequencing for efficient screening of DNA sequence variation" CLINICAL CHEMISTRY, Bd. 42, Nr. 9, 1996, Seiten 1391-1397, XP002116875
- SHI M.M.: "Enabling large-scale pharmacogenetic studies by high-throughput mutation detection and genotyping technologies" CLINICAL CHEMISTRY, AMERICAN ASSOCIATION FOR CLINICAL CHEMISTRY. WINSTON, US, Bd. 47, Nr. 2, Februar 2000 (2000-02), Seiten 164-172, XP002197957 ISSN: 0009-9147

## Beschreibung

Die vorliegende Erfindung betrifft ein Verfahren zum multiparallelen Nachweis von Nukleotidpolymorphismen auf einem polydimensionalen Microarray. Des Weiteren betrifft die Erfindung ein Verfahren zur Herstellung polydimensionaler Microarrays für den Nachweis vieler einzelner Nukleotidpolymorphismen, wobei die Nukleotidpolymorphismen multipler Individuen auf dem Microarray multiparallel nachgewiesen werden können.

Bereits seit einigen Jahren werden natürliche DNA-Variationen zwischen Individuen in den Bereichen Pharmacogenomics und Pharmacogenetics sowie allgemein zur Genotypisierung menschlicher, tierischer, mikrobieller, pilzlicher und pflanzlicher Individuen untersucht. Eine Form von Variationen in DNA-Sequenzen, die die DNA eines Individuums von der eines anderen Individuums unterscheiden, stellen die so genannten singulären Nukleotidpolymorphismen (SNPs, single nucleotide polymorphisms) dar, die auch zwischen Individuen der gleichen Spezies existieren.

Ein SNP ist ein einzelner DNA-Basenaustausch oder eine Insertion/Deletion einzelner Basen in einer bestimmten Position innerhalb eines genomischen Abschnitts wie z.B. einem Gen. Zum Beispiel können einige Individuen in einer Population die Base Adenin aufweisen, während andere Individuen an der gleichen Stelle innerhalb eines Gens die Base Cytosin besitzen. Betrachtet man mehrere dieser SNPs in einem Individuum, so bietet dies die Möglichkeit, einen genetischen Fingerabdruck für das jeweilige Individuum zu erstellen. Diese Unterschiede können mittels speziell für die SNP-Analyse ausgerichteter DNA-Microarrays untersucht und ausgewertet werden. Die Analyse von SNPs ist allgemein zur Beantwortung molekulargenetischer Fragestellungen nützlich. Zum Beispiel kann das Ergebnis einer SNP-Analyse wertvolle Informationen über die Prädisposition eines Individuums für eine bestimmte Erkrankung liefern. Auf diese Weise ist es möglich, vor der Medikation eines Patienten abzuschätzen, wie der Patient auf eine bestimmte Wirkstoffklasse reagieren wird.

Die SNP-Analyse, häufig auch als Genotypisierung bezeichnet, ist aber nicht nur für die medizinische Genetik und die Pharmakogenetik von besonderem Interesse, die Feststellung, ob ein bestimmter SNP und damit eine bestimmte Eigenschaft in einem Individuum vorhanden oder abwesend ist, ist allgemein für die Charakterisierung von Individuen, seien sie menschlicher, tierischer, pflanzlicher Natur oder Mikroorganismen, nützlich. So ist die SNP-Genotypisierung bereits seit einigen Jahren ein fester Bestandteil der modernen Pflanzenzüchtung.

Die SNP-Analyse wird inzwischen besonders im Bereich der Biomedizin routinemäßig durchgeführt und von entsprechend ausgerichteten Firmen als Serviceleistung angeboten. Auch sind bereits zur SNP-Analyse geeignete Reaktionskits verschiedener Anbieter auf dem Markt erhältlich, allerdings nicht für Microarrays. Dabei werden vorwiegend zwei Methoden zum Nachweis der Punktmutationen auf einem DNA-Chip bzw. Microarray eingesetzt, dies sind zum einen die Allel-spezifische Hybridisierung, zum anderen die Primer-Extension. Diese Nachweismethoden basieren auf Oligonukleotiden, die auf dem Chip in Form eines so genannten Arrays, also einer vorbestimmten Anordnung, angeordnet sind, um einzelne Basenpolymorphismen in einer zu untersuchenden DNA-Probe durch entweder Hybridisierung oder Hybridisierung gefolgt durch eine DNA-Polymeraseabhängige Primer-Extension der angeordneten Oligonukleotide nachzuweisen. Bei beiden Techniken ist die Sonde, also das jeweilige Oligonukleotid auf dem Chip in einer bestimmen Position angeordnet und fixiert, während die zu untersuchenden Nukleinsäuremoleküle der Probe in Form einer Hybridisierungslösung vorliegen. Diese Lösung wird mit dem Chip in Kontakt gebracht und inkubiert, wodurch die in der Lösung enthaltenen DNA-Moleküle der Probe ihre passenden Hybridisierungspartner, nämlich die zu dem jeweiligen Molekül passende Oligonukleotidsonde, auf der Oberfläche des Biochips finden und mit dieser hybridisieren.

Die Begriffe DNA-Chip, Biochip und Microarray werden häufig austauschbar untereinander verwendet, so auch in der vorliegenden Anmeldung. Streng genommen bedeutet Microarray lediglich, dass Moleküle an bestimmten Positionen innerhalb einer Anordnung (Array) oder Rasters in einer hohen Dichte angeordnet sind. Tatsächlich können Microarrays bis zu mehrere Hunderttausend Positionen (häufig als Spots bezeichnet) auf einem Träger oder einer Matrix aufweisen. Der Chip ist das eigentliche Arraysubstrat, also der Träger eines oder in der Regel vieler Microarrays. Als Träger werden u.a. Objektträger oder andere Substrate aus Glas und Wafer verwendet.

Bei der Genotypisierung, also dem Nachweis der Anwesenheit oder Abwesenheit eines SNP unter Einsatz der gegenwärtig üblichen Microarray-Technologien müssen in der Regel zahlreiche DNA-Moleküle bzw. Loci eines einzelnen Individuums ihre verschiedenen Hybridisierungspartner finden. Es ist ersichtlich, dass hieraus einige Probleme entstehen, insbesondere mit steigender Anzahl der zu untersuchenden Loci. Häufig finden Probenmoleküle ihre passenden Sonden nicht, insbesondere wenn das Experiment komplex ist und viele Probenmoleküle in der Lösung enthalten sind und viele immobilisierte Oligonukleotidsonden sich auf der Chipoberfläche befinden, z.B. 1000 Sonden oder mehr. Hier ist das Hybridisierungsergebnis häufig schlecht, und viele Probenmoleküle finden niemals ihren Sondenpartner, was zwangsläufig zu fehlenden Signalen und falschen Ergebnissen führt. Des Weiteren müssen die Probenmoleküle bzw. zu untersuchenden Loci von ein und demselben Individuum stammen, da bei der Verwendung von zwei oder mehr Individuen bzw. deren Proben automatisch Kreuzhybridisierung der homologen Probenmoleküle mit der gleichen Sonde stattfinden würde und die Signale somit nicht mehr unterscheidbar und zuordenbar wären.

Ähnliche Probleme treten nicht nur bei dem parallelen Nachweis mehrerer SNPs von vielen Individuen auf, sondern auch bei allen anderen Microarray-basierten Verfahren, die auf vielen parallelen Hybridisierungsereignissen zwischen Sonden und Targets beruhen. Die gilt insbesondere beim Nachweis ähnlicher Sequenzen.

Ein typisches Beispiel für solche Verfahren ist die Verwendung von Microarrays zum Nachweis von Mikroorganismen in Proben in der biomedizinischen Diagnostik. Dabei macht man sich die Tatsache zunutze, dass die Gene für ribosomale RNA (rRNA) ubiquitär verbreitet sind und über Sequenzabschnitte verfügen, die für die jeweilige Spezies charakteristisch sind. Diese Spezies-charakteristischen Sequenzen werden in Form von einzelsträngigen DNA-Oligonukleotiden auf ein Microarray aufgebracht. Die zu untersuchenden Target-DNA-Moleküle werden zunächst aus der zu untersuchenden Probe isoliert und mit fluoreszierenden Markern versehen. Anschließend werden die markierten Target-DNA-Moleküle in einer Lösung mit den auf den Microarray aufgebrachten Sonden inkubiert, unspezifisch auftretende Wechselwirkungen werden durch entsprechende Waschschritte entfernt und spezifische Wechselwirkungen durch fluoreszenzoptische Auswertung nachgewiesen. Auf diese Art und Weise ist es möglich mit einem einzigen Test in einer Probe gleichzeitig z. B. mehrere Mikroorganismen nachzuweisen. Die Anzahl der nachweisbaren Mikroorganismen hängt bei diesem Testverfahren theoretisch nur von der Anzahl der spezifischen Sonden ab, die auf dem Microarray aufgebracht worden sind. Da jedoch häufig Mikroorganismen mit sehr ähnlichen rRNA-Sequenzen parallel nachgewiesen werden sollen, treten die für die SNP-Analyse beschriebenen Probleme, die auf Kompetition, Kreuzhybridisierung etc. beruhen, auch hier auf.

Die technischen Möglichkeiten, die genannten Nachteile durch den Einsatz verschiedener Laser und Fluorchrome zu kompensieren, sind sehr begrenzt. Im Ergebnis bedeutet dies, dass z.B. bei der SNP-Analyse für die Genotypisierung jedes Einzelindividuums ein separater Chip hergestellt und eingesetzt werden muss. So auch kürzlich beschrieben in Science (2002), 295, Seiten 160 - 172, wo ein aktueller Überblick über DNA-Chip- und Microarray-Technologien gegeben wird; siehe hier insbesondere Seite 172, 3. Spalte. Die Herstellung individueller Microarrays für jedes zu untersuchende Individuum ist derart zeit- und kostenintensiv, dass diese Vorgehensweise zum Beispiel in einer Studie mit 5000 Testindividuen, die angesichts der Notwendigkeit von 5000 SNP-Chips allein für den Hybridisierungs-Nachweisschritt fast 5 Mio. EUR kosten würde, kaum durchführbar ist.

Obwohl SNPs und die Genotypisierung von Patienten besonders in der medizinischen Genetik von zentraler Bedeutung sind, wurden bis heute keine praktikablen Ansätze entwickelt, die die Nachteile des Standes der Technik überwinden und die multiparallele Analyse vieler Loci von vielen Individuen gleichzeitig ermöglichen.

Aufgabe der Erfindung ist es daher, die parallele Analyse mehrerer Nukleinsäuren durch Hybridisierung gleichzeitig und vergleichbar auf einem Microarray möglich zu machen, wobei die Fehlerquellen möglichst eingeschränkt sein sollen und keine hohen Anforderungen an die Analyseparameter wie z.B. Probenkonzentration gestellt werden.

Aufgabe der Erfindung ist es daher ebenfalls, die Analyse vieler diagnostischer Mutationen oder SNPs an vielen Loci gleichzeitig und vergleichbar auf einem Microarray möglich zu machen. Dabei sollen möglichst keine hohen Anforderungen an die verschiedenen Analyseparameter, wie gleiche Hybridisierungstemperatur oder DNA-Probenkonzentration auf dem Microarray, bestehen. Vielmehr soll ein möglichst einfaches und zuverlässiges Nachweisverfahren mit möglichst wenigen Fehlerquellen bereitgestellt werden. Aus diesem Grund soll auch auf ein kompliziertes zyklisches Temperaturregime, wie es etwa bei der PCR notwendig ist, verzichtet werden.

Diese und andere Aufgaben der Erfindung, wie sie sich aus der Beschreibung ergeben, werden durch den Gegenstand des unabhängigen Anspruchs gelöst.

Bevorzugte Ausführungsformen der Erfindung sind in den Unteransprüchen definiert.

Die genannten Aufgaben werden durch das erfindungsgemäße Verfahren zur gleichzeitigen Genotypisierung multipler Individuen und multipler Loci auf ein und dem selben Träger (DNA-Chip bzw. Microarray) gelöst.

Die Methoden des Standes der Technik zum Microarray-basierten Nachweis von Nukleinsäuren verlangen allgemein, dass die Proben- bzw. Targetmoleküle in der Hybridisierungslösung gepoolt werden und die Hybridisierungslösung mit dem Chip, auf dem die Oligonukleotidsonden angeordnet sind, in Kontakt gebracht wird, was dazu führt, dass die verschiedenen Probenmoleküle ihren Hybridisierungspartner unter zahlreichen Sonden, insbesondere bei steigender Anzahl der analysierten Probenmoleküle, finden müssen. Die gleichen Probleme würden sich auch ergeben, wenn anstelle der Sonden- die Probenmoleküle immobilisiert und mit mehreren Sondenmolekülen in Kontakt gebracht würden. Im Unterschied hierzu basiert die Erfindung darauf, dass nicht zuerst Sondenmoleküle in diskreten Positionen auf dem Chip angeordnet werden, um im zweiten Schritt zwecks Hybridisierung mit den Probe- bzw. Targetmolekülen in Kontakt gebracht zu werden, sondern vielmehr der Schritt der Bildung von Hybriden aus Sondenmolekül und Probenmolekül zeitlich vor der Positionierung und Immobilisierung auf dem Chip erfolgt. Im Rahmen des erfindungsgemäßen Verfahrens findet also zuerst die Hybridisierung statt und anschließend die Anordnung der Sonden/Proben-Hybride auf dem Chip bzw. Microarray.

US 5,759,777 A offenbart ebenfalls die Immobilisierung von Hybriden, allerdings findet in diesem Verfahren die Hybridisierung nicht zwischen einem Sondenmolekül und einem Probenmolekül statt, sondern zwischen einem Sondenmolekül und einem Extrakt, in dem sich viele verschiedene Probenmoleküle befinden. Dadurch ergeben sich die bereits vorher diskutierten Probleme der Kreuzhybridisierung.

Bei dem Nachweis von SNPs verlangen die Methoden des Stands der Technik ebenfalls, dass die Probenmoleküle eines Einzelindividuums in der Hybridisierungslösung gepoolt werden und die Hybridisierungslösung mit dem Chip, auf dem die Oligonukleotidsonden angeordnet sind, in Kontakt gebracht wird, was dazu führt, dass verschiedene Probenmoleküle eines Individuums ihren Hybridisierungspartner unter zahlreichen Sonden, insbesondere bei steigender Anzahl der analysierten Loci, finden müssen. Im Unterschied hierzu basiert die Erfindung darauf, dass nicht zuerst Sondenmoleküle in diskreten Positionen auf dem Chip angeordnet werden, um im zweiten Schritt zwecks Hybridisierung mit den Probe- bzw. Targetmolekülen des zu untersuchenden Individuums in Kontakt gebracht zu werden, sondern vielmehr der Schritt der Bildung von Hybriden aus Sondenmolekül und Probenmolekül zeitlich vor der Positionierung und Immobilisierung auf dem Chip erfolgt. Im Rahmen des erfindungsgemäßen Verfahrens findet also zuerst die Hybridisierung statt und anschließend die Anordnung der Sonden/Proben-Hybride auf dem Chip bzw. Microarray.

Im Stand der Technik findet die Immobilisierung der Proben- oder Sondenmoleküle im Rahmen des SNP-Nachweises häufig an Kügelchen durch Wechselwirkung mit der magnetischen Ladung oder mit funktionellen Gruppen der Kügelchen statt. Solche Verfahren ermöglichen es nicht, viele Analysen reagenzien- und zeitsparend parallel durchzuführen. Dagegen findet die Immobilisierung der Hybride im Rahmen der vorliegenden Erfindung auf einem Microarray bzw. Chip statt, was eine große Zeit- und Reagenzienersparnis darstellt.

Durch die erfindungsgemäße Immobilisierung bereits geformter Hybride aus Sondenmolekül und Probenmolekül entfällt das Problem der Verfahren des Standes der Technik, das daraus resultiert, dass bei herkömmlichen Verfahren jedes Probenmolekül seine immobilisierte Partnersonde unter vielen anderen Molekülen finden muss. Diese Fehlerquelle, die die oben beschriebenen Nachteile und Risiken birgt und den Verfahren des Standes der Technik somit deutliche Grenzen setzt, wird gemäß der vorliegenden Erfindung eliminiert.

Bei einer bevorzugten Ausführungsform der vorliegenden Erfindung zum SNP-Nachweis findet die Bildung des Hybrids aus Sondenmolekül und Probenmolekül individuell für jeden zu untersuchenden Locus und jedes zu untersuchende Individuum statt. Hierbei kann die Hybridisierung zum Beispiel in Mikrotiterplatten stattfinden, wobei natürlich auch andere Träger, Gefäße oder Behältnisse als Ort der Hybridisierung einsetzbar sind. Wichtig ist nur, dass die Oligonukleotidsonden separat vorliegen und festgelegt ist, welche Oligonukleotidsonde sich in welchem Napf der Mikrotiterplatte bzw. einem anderen Reaktionsgefäß befindet, da die sichere Zuordnung des jeweiligen Oligonukleotids zu der späteren Position des Hybrids aus Sonde und Probe auf dem Chip sicher gestellt sein muss.

Aus technischen Gründen ist es sinnvoll, die Oligonukleotidsonden in einer vorbestimmten Anordnung innerhalb einer Mikrotiterplatte vorzugeben, so dass sich aus der Anordnung der Sondenoligonukleotide in der Mikrotiterplatte unmittelbar die Anordnung der späteren Hybride auf dem Chip ergibt.

Nachdem sich die durch die Kenntnis der Oligonukleotidsonde definierten Hybride aus Oligonukleotidsonde und Probenmolekül geformt haben, werden diese auf dem Chip immobilisiert und anschließend durch Primer-Extension verlängert.

Dem Fachmann ist klar, dass das erfindungsgemäße Verfahren zum SNP-Nachweis prinzipiell auch auf andere Microarray-basierte Nachweisverfahren ausgedehnt werden kann. Z.B. können erfindungsgemäß zum Nachweis von Mikroorganismen in einer Probe ebenfalls die durch PCR amplifizierten Targetmoleküle zunächst z.B. in Mikrotiterplatten in festgelegten Anordnungen mit jeweils einer Mikroorganismusspezifischen rDNA-Sonde hybridisiert werden. Nach der Hybridisierung und Immobilisierung auf dem Chip erfolgt dann die Auswertung. Auf diese Weise können Kompetitionsreaktionen von verschiedenen Sonden um Targets in einfacher Weise minimiert werden. Da nur eine Sonde zur Hybridisierung zur Verfügung steht, kann die Hybridisierung zudem effizienter durchgeführt werden, was zu besseren Signal-Rausch-Verhältnissen bei der Auswertung führt. Zudem entfallen Probleme, die durch die gleichzeitige Diffusion vieler Targets zu vielen Sonden bei Auswertungen nach dem Stand der Technik auftreten.

Bei anderen Anwendungen kann es sich z.B. um die Analyse der transkriptionellen Aktivität eines Organismus oder einer Zelle handeln. In diesem Fall wird durch Reverse Transkription zunächst die mRNA, d.h. die exprimierte genetische Information, quantitativ in entsprechende cDNAs überführt. Nach der Amplifikation dieser cDNAs kann dann in einem erfindungsgemäßen Verfahren durch Hybridisierung jeweils einer amplifizierten cDNA mit jeweils einer Sonde, Immobilisierung des Hybrids und Auswertung der Hybridisierung auf die Genaktivität der jeweiligen Zelle etc. geschlossen werden.

Dem Fachmann sind weitere Anwendungen des erfindungsgemäßen Verfahrens, wie z.B. Mutationsanalysen, Rekombinationsanalysen, Stammbaumanalysen und Segregationsanalysen bekannt.

Die Erfindung betrifft somit ein Verfahren zum Nachweis von Nukleinsäuren, umfassend die nachfolgenden Schritte in der angegebenen Reihenfolge:
a) Mischen und Hybridisieren eines ersten einzelsträngigen Nukleinsäuremoleküls (Probenmolekül) und eines zweiten einzelsträngigen Nukleinsäuremoleküls (Sondenmolekül), dessen Sequenz zu der des ersten Nukleinsäuremoleküls mindestens teilweise komplementär ist, so dass das erste und zweite Nukleinsäuremolekül mindestens über die komplementäre Sequenz miteinander hybridisieren können,
b) Anordnen und Immobilisieren des gebildeten Hybrids aus erstem und zweitem Nukleinsäuremolekül auf einem Träger, und
c) Nachweis der Hybridisierungsreaktion mit einem geeigneten Nachweisverfahren.

Das Verfahren der Erfindung eignet sich zum parallelen Nachweis von z.B. mehreren Probenmolekülen in einer Mischung, zum parallelen Nachweis von mehreren Organismen, bevorzugt Mikroorganismen in einer Probe, zum Analysieren der Genaktivität mehrerer Gene eines Organismus oder einer Zelle etc.

Die Erfindung betrifft in einer bevorzugten Ausführungsform ein Verfahren zum Nachweis eines Nukleotidpolymorphismus (SNP), umfassend die nachfolgenden Schritte in der angegebenen Reihenfolge:
a) Mischen und Hybridisieren eines ersten einzelsträngigen Nukleinsäuremoleküls (Probenmolekül), das den nachzuweisenden SNP aufweist, und eines zweiten einzelsträngigen Nukleinsäuremoleküls (Sondenmolekül), dessen Sequenz zu der des ersten Nukleinsäuremoleküls mindestens teilweise komplementär ist, so dass das erste und zweite Nukleinsäuremolekül mindestens über die komplementäre Sequenz miteinander hybridisieren können,
b) Anordnen und Immobilisieren des gebildeten Hybrids aus erstem und zweitem Nukleinsäuremolekül auf einem Träger, und
c) Nachweis des SNPs über Auswertung der Hybridisierungsreaktion mit einem geeigneten Nachweisverfahren.

Das Verfahren der Erfindung eignet sich damit zum Nachweis bzw. zur Genotypisierung der Anwesenheit, Abwesenheit oder Identität eines einzelnen Nukleotidpolymorphismus in einer bestimmten Position innerhalb des Genoms eines Individuums. Bevorzugt werden mehrere bis viele Individuen gleichzeitig auf einem gemeinsamen Träger bezüglich einer bestimmten Nukleotidvariation untersucht.

Besonders bevorzugt werden mehrere bis viele Individuen gleichzeitig auf einem gemeinsamen Träger bezüglich mehrerer bis vieler Nukleotidvariationen untersucht. Das Verfahren der Erfindung eignet sich ebenfalls zum Nachweis bzw. zur Genotypisierung der Anwesenheit, Abwesenheit oder Identität von Insertionen oder Deletionen einer oder mehrerer Basen in einer bestimmten Position innerhalb des Genoms eines Individuums.

Die Erfindung betrifft weiterhin ein Verfahren zur Herstellung von Microarrays zur multiparallelen SNP-Analyse mehrerer Individuen und mehrerer Loci im Genom eines Individuums, umfassend die nachfolgenden Schritte in der angegebenen Reihenfolge:
a) Mischen und Hybridisieren eines ersten einzelsträngigen Nukleinsäuremoleküls (Probenmolekül), das einen nachzuweisenden SNP aufweist, und eines zweiten einzelsträngigen Nukleinsäuremoleküls (Sondenmolekül), dessen Sequenz zu der des ersten Nukleinsäuremoleküls mindestens teilweise komplementär ist und einen bestimmten Locus im Genom eines Individuums repräsentiert, so dass das erste und zweite Nukleinsäuremolekül mindestens über die komplementäre Sequenz miteinander hybridisieren können,
b) Anordnen und Immobilisieren des gebildeten Hybrids aus erstem und zweitem Nukleinsäuremolekül, das den besagten Locus im Genom eines Individuums repräsentiert, auf einem Träger, und
c) enzymatische Template-abhängige Extension des zweiten Nukleinsäuremoleküls, das innerhalb des Hybrids als Primer fungiert, unter Einbau eines zum Kettenabbruch führenden Nukleotids oder -analogons,
wobei Sondenmoleküle, die jeweils verschiedene Loci repräsentieren, und Probenmoleküle mehrerer Individuen zur parallelen Analyse verwendet werden.

Erfindungsgemäß können auch Sondenmoleküle, die jeweils verschiedene Loci jeweils verschiedener Individuen repräsentieren, zur parallelen Analyse verwendet werden.

Verschiedene Ausführungsformen des erfindungsgemäßen Verfahrens zur Herstellung von Microarrays zur multiparallelen SNP-Analyse mehrerer Individuen und mehrerer Loci im Genom eines Individuums sind in den Unteransprüchen definiert.

Unter "Locus" wird erfindungsgemäß ein genomischer Abschnitt verstanden, in dem der zu untersuchende genetische Polymorphismus, wie z.B. der SNP, die Insertion oder Deletion, liegt.

Als "Immobilisieren" wird im Rahmen der vorliegenden Erfindung der Vorgang bezeichnet, durch den ein Molekül auf einem festen Träger durch kovalente oder nicht-kovalente Wechselwirkung derart angebracht wird, dass das Molekül sich nicht mehr frei auf dem Träger bewegen bzw. von dem Träger weg in Lösung diffundieren kann.

Als "Träger", "Matrix" oder "Substrat" werden im Rahmen der vorliegenden Erfindung solche Vorrichtungen bezeichnet, auf denen Moleküle durch kovalente oder nicht-kovalente Wechselwirkungen aufgebracht werden können. Als Träger bzw. Substrat für Nukleinsäuremoleküle kommen z.B. Objektträger oder andere Materialien aus Glas sowie Wafer in Frage.

Erfolgt die Lokalisierung auf dem Träger, der Matrix oder dem Substrat in einer definierten räumlichen Anordnung, so wird diese Anordnung häufig auch als Array bezeichnet. Eine spezifische Position des Arrays wird üblicherweise auch als Spot bezeichnet. Dem Fachmann ist klar, dass die Begriffe "Array", "Substrat", "Matrix" etc. im Stand der Technik häufig synonym füreinander verwendet werden und voneinander durch Definitionen nicht strikt getrennt werden können.

Der Begriff "Chip", "DNA-Chip" oder "Gen-Chip" bezeichnet üblicherweise die Anordnung eines Moleküls im Arrayformat auf einem Träger.

Der Nachweis eines SNPs in einem mit der Sonde hybridisierten und auf einem Träger immobilisierten Probenmolekül kann durch verschiedene dem Fachmann geläufige Nachweisverfahren erfolgen. Das etablierteste und am häufigsten verwendete Verfahren zum Nachweis von SNPs in Proben-Sonden-Hybriden ist die so genannte Primer-Extension.

Die Primer-Extension, also die Verlängerung des als Primer dienenden Sondenmoleküls in Template-abhängiger Weise, wobei das Probenmolekül das Template darstellt, kann auf herkömmliche Weise erfolgen, wie zum Beispiel beschrieben in EP 0 648 280 B1, EP 0 705 349 B1 und WO 98/59066 A1. Auf die in diesen Dokumenten enthaltene Offenbarung zur Primer-Extension wird hiermit ausdrücklich verwiesen.

Bei der für die SNP-Analyse inzwischen üblich gewordenen Methode der Primer-Extension werden Primermoleküle, die eine zu einer oder mehreren Nukleotidsequenzen eines genomischen DNA-Segments eines Individuums komplementäre Polynukleotidsequenz aufweisen, wobei das genomische Segment unmittelbar 3'-distal zu einem SNP, X, lokalisiert ist, durch Template-abhängige Extension des Nukleinsäureprimermoleküls durch ein einziges Nukleotid oder Nukleotidanalogon, das zu dem Nukleotid X des SNP-Allels komplementär ist, verlängert. Findet die Template-abhängige Extension des Primers, der in der Technik häufig als Interrogations-Primer bezeichnet wird, in Anwesenheit von einem oder mehreren Didesoxynukleotidtriphosphat-Derivaten oder -Analoga, ausgewählt aus der Gruppe bestehend aus ddATP, ddTTP, ddCTP und ddGTP, oder anderen zum Kettenabbruch führenden Basenanaloga, aber in Abwesenheit von dATP, dTTP, dCTP und dGTP statt, so kann das nicht verlängerbare Didesoxynukleotidtriphosphat-Derivat in der Position des SNP und somit der SNP selbst auf herkömmliche Weise nachgewiesen werden. Wie oben erwähnt, ist der Schritt der Primer-Extension, die den SNP-Nachweis unmittelbar ermöglicht, im Stand der Technik vielfach beschrieben und kann vom Fachmann auch mittels hierfür auf dem Markt erhältlicher Reagenzien und Reaktionskits durchgeführt werden.

Dabei stellt das Oligonukleotid, das als Sondenmolekül für die Bildung des Hybrids aus Sondenmolekül und Probenmolekül eingesetzt wird, in der Regel auch den Primer dar, der durch eine Polymerase Template-abhängig um das in Rede stehende Nukleotid, das dem SNP-Allel entspricht, verlängert wird.

Bei anderen, dem Fachmann bekannten Nachweisverfahren zur Detektion der SNPs im Hybrid handelt es sich z.B. um die Allel-spezifische Primer-Extension (siehe unten), um Allel-spezifische Hybridisierung (siehe unten) und die Massenspektroskopie.

Bedingt durch die zeitliche Abfolge der Schritte Hybridisierung und Anordnung bzw. Immobilisierung auf dem Träger bzw. Chip gemäß der Erfindung, nämlich erstens Bildung des Hybrids und anschließende Immobilisierung des Hybrids auf dem Chip, werden die auf dem Chip angeordneten und immobilisierten Hybride aus Sondenmolekül und Probenmolekül zu keinem Zeitpunkt vor der Extension denaturiert und stellen vielmehr unmittelbar das Substrat für die Polymerase dar, die ein nachweisbares, in der Regel markiertes Nukleotid an das in Rede stehende Sondenmolekül anfügt. Da somit die zu untersuchenden Probenmoleküle zusammen mit der Sonde auf dem Chip immobilisiert werden, und nicht, wie im Stand der Technik, in einer Hybridisierungslösung vorliegen, ist jegliche Kreuzhybridisierung ausgeschlossen. Hieraus bietet sich erstmals die Möglichkeit, verschiedene Hybride aus Sondenmolekül und Probenmolekül, die verschiedene Positionen innerhalb eines Genoms, also verschiedene Loci repräsentieren, von verschiedenen Individuen, sogar von verschiedenen Spezies oder Populationen, parallel auf einem einzigen Chip zu analysieren. Es können dank der vorliegenden Erfindung somit erstmals mehrere Loci von mehreren Individuen parallel auf ein und demselben Chip zuverlässig untersucht und ausgewertet werden.

Während die Analyse mehrerer diagnostischer Mutationen oder SNPs an vielen Loci gleichzeitig und vergleichbar auf einem Microarray bisher nur durch serielle Analyse, entweder einzelner Proben, also jeweils ein Individuum und ein SNP in einem Arbeitsgang, oder eingeschränkt parallel, also entweder ein Individuum mit mehreren Loci oder ein Locus mit mehreren Individuen in einem Arbeitsgang bzw. einem Reaktions- oder Hybridisierungsraum, möglich war, können jetzt erstmals viele Individuen parallel bezüglich verschiedener Loci effizient, kostengünstig und reproduzierbar in einem Experiment auf einem einzigen Chip analysiert werden.

Dies ist möglich, weil bei einer bevorzugten Ausführungsform des erfindungsgemäßen Verfahrens jeder zu untersuchende Locus jedes Individuums einzeln durch PCR amplifiziert und mit jeweils einer für einen Locus charakteristischen Sonde vor der Immobilisierung hybridisiert wird.

Das erfindungsgemäße Verfahren zum Nachweis von SNPs bzw. zur Herstellung multiparalleler SNP-Microarrays wird im Folgenden hinsichtlich der einzelnen Schritte und Hybridisierungspartner genauer beschrieben.

Bei der Probennukleinsäure, hier auch Target-Nukleinsäure oder Ziel-Nukleinsäure genannt, von der man vermutet, das sie den variablen Nukleotidrest, also den SNP, enthält und die deshalb hierauf hin analysiert werden soll, kann es sich um eine menschliche, tierische, pflanzliche, pilzliche oder mikrobielle Nukleinsäure (DNA oder RNA) handeln. Dabei kann die Probennukleinsäure aus biologischen Proben mittels konventioneller Nukleinsäurereinigungsmethoden isoliert werden, oder auch in ungereinigter Form innerhalb einer biologischen Probe vorliegen.

Einzelne DNA-Fragmente, die vermutlich einen variablen Nukleotidrest, einen SNP, tragen, werden mit entsprechenden PCR-Primern aus einer isolierten (aufgereinigten) DNA oder RNA amplifiziert oder auch direkt aus der nicht extra aufgereinigten biologischen Probe (z.B. Zellsaft oder Quetschpräparat; sog. Rohextrakte) amplifiziert. Dies ist möglich, wenn die Probe sog. DNA-fähiges Material enthält. Dem Fachmann ist bekannt, dass RNA durch Reverse Transkription zunächst in eine cDNA umgeschrieben werden kann, die dann wieder als Template in z.B. einer PCR dienen kann.

Die Methoden zur enzymatischen Anreicherung von Nukleinsäuren (wie z.B. PCR) und deren spezifische Ausführungsformen sind dem Fachmann wohl vertraut und gut in der Literatur dokumentiert (Sambrook et al. (2001), Molecular Cloning: A laboratory manual, 3rd edition, Cold Spring Harbor Laboratory Press).

Es können aber auch PCR-unabhängige Anreicherungsschritte durchgeführt werden, die z.B. auf Affinitätschromatographie oder magnetischen Mikropartikeln (z.B. Dynabeads^{™}) basieren. Proben, die nicht aufgereinigt, angereichert oder amplifiziert werden, können unter Umständen auch verwendet werden, benötigen später aber einen Verstärkungsschritt der Fluoreszenzsignale (Signal-Amplifikation).

Zur effektiveren Hybridisierung von Proben- und Sondenmolekül ist es vorteilhaft, wenn das Probenmolekül einzelsträngig ist. Dies wird erreicht, indem einer der beiden PCR-Primer (und zwar der Primer, der den Strang synthetisiert, der zur Hybridisierung genutzt wird) eine chemische Modifikation trägt, die ihn davor schützt, von einer 5'-Exonuklease abgebaut zu werden. Ist diese Modifikation am ersten 5'-Nukleotid des Primers eingefügt, wird dieser Strang nicht abgebaut, während der gesamte komplementäre Gegenstrang, der zur Hybridisierung mit dem Sondenmolekül nicht benötigt wird, vom Primer aus in 5'-3'-Richtung von einer 5'-Exonuklease abgebaut, also eliminiert, wird. Es ist auch möglich, ein entsprechendes System mit 3'-Exonukleasen zu entwickeln.

Gängige Primer-Modifikationen sind z.B. 5'-PTO (5'-Phosphoro-Thioat-Nukleotide), bei denen die Phosphatgruppen 5'-endständiger Nukleotide des Primers durch Phosphoro-Thioat-Gruppen ersetzt sind. Solche modifizierten Primer werden durch die 5'-Exonuklease des Phagen T7 (Gen 6) oder die 5'-Exonuklease des Lambda Phagen nicht abgebaut. Aber auch andere 5'-Schutzgruppen oder PNA (Peptide Nucleic Acid) Primer können zum Schutz des hybridisierenden Strangs verwendet werden.

Eine weitere Methode zur Herstellung weitgehend einzelsträngiger Probenmoleküle, die nicht auf Modifikation gefolgt von enzymatischem Abbau basiert, ist die sog. "asymmetrische PCR". Hier wird der Primer des benötigten Strangs in vielfachem Überschuss zum Primer des nicht benötigten Strangs hinzugegeben. Dies führt zur bevorzugten Synthese des für die Hybridisierung benötigten Strangs. Die Hybridisierung wird durch den im wesentlichen nicht amplifizierten Gegenstrang nicht behindert.

Einzelsträngige Probenmoleküle können aber auch durch Anreicherungsmethoden mit magnetischen Partikeln oder Affinitätschromatographie erhalten werden. Diese Substrate enthalten der Zielsequenz komplementäre Nukleotide auf ihrer Oberfläche und fischen die gewünschten Sequenzen aus einer denaturierten, fragmentierten genomischen DNA heraus und reichern sie an (z.B. das *Kingfischer-*System von Hybaid).

Bei den Sondenmolekülen handelt es sich um Oligo- oder Polynukleotide, die aufgrund ihrer Nukleotidsequenz mit in der Probe vermuteten Probenmolekülen hybridisieren können. Bei den Molekülen kann es sich um DNA oder RNA handeln.

Die Sondenmoleküle können nach im Stand der Technik wohl bekannten Techniken hergestellt werden. Sie können auch von Anbietern bezogen werden, die Oligo- und Polynukleotide kommerziell herstellen.

Bei den Sondenmolekülen handelt es sich üblicherweise um künstlich synthetisierte einzelsträngige Oligonukleotide, die, wenn als Nachweisverfahren die Primer-Extension verwendet wird, mit ihrem letzten 3'-Nukleotid direkt vor der polymorphen Position in der Sequenz des zu untersuchenden Individuums enden. Sie sind komplementär zum 5'-modifizierten PCR-Primer der Einzelstrangprobe, um das Zusammenpassen von Sonde und Probe zu garantieren. Weiterhin tragen die Extensionsprimer an ihrem 5'-Ende, mit dem sie an die Chipoberfläche gebunden werden, einen sog. *Spacer* (Platzhalter; meist 6 bis 24 C-Atome; aber auch polyA- oder polyT-Spacer sind bekannt), an dessen vom Primer abgewandten Ende sich eine Amino-Modifikation befindet.

Diese Aminogruppe (der sog. *Linker*) reagiert unter UV-Bestrahlung mit der Epoxy-Beschichtung des Chips und bindet somit das Sondenmolekül, oder in diesem Fall die Hybridstruktur aus Sonde und Probe, chemisch kovalent an die Beschichtung.

Dem Fachmann sind weitere funktionelle Gruppen bekannt, die zur Kopplung der Sonde bzw. des Hybrids aus Sonde und Target an die Array-Oberfläche verwendet werden können (siehe unten).

Wie oben dargestellt, stellen die Sondenmoleküle in einer Ausführungsform der Erfindung die so genannten Interrogations-Primer, im Stand der Technik auch Nachweisschritt-Primer genannt, dar, die durch Primer-Extension durch die Aktivität einer Polymerase in Template-abhängiger Weise verlängert werden. In diesem Fall ist der Primer, also das Sondenmolekül, komplementär zu der Nukleotidsequenz 3' des variablen Nukleotids (SNP) im dazu gehörigen Probenmolekül. Der Primer, der somit als Startpunkt für die Template-abhängige Elongation durch eine DNA-Polymerase fungiert, wird derart ausgewählt, dass er mit einer Nukleotidsequenz unmittelbar neben oder in der Nähe des variablen Nukleotids, das im Rahmen der SNP-Analyse nachzuweisen ist, hybridisiert. Dabei kann der Primer, also das Sondenmolekül, so gewählt werden, dass er komplementär zu entweder dem kodierenden oder dem nicht-kodierenden Strang eines doppelsträngigen Target-Moleküls ist, in Abhängigkeit davon, welcher Strang in dem Hybrid, aus Sondenmolekül und Probenmolekül, das nach erfolgter Hybridisierung auf dem Chip immobilisiert wird, vorliegen soll.

Die Auswahl des Sondenmoleküls, das zugleich der Interrogations-Primer ist, wird durch die Natur der Nukleotidvariation, die analysiert werden soll, bestimmt. Dabei wird der Interrogations-Primer in einer Ausführungsform der Erfindung derart gewählt und hergestellt, dass er unmittelbar neben dem nachzuweisenden variablen Nukleotid liegt, wenn sich das Hybrid aus Primer und Probenmolekül gebildet hat.

In einer anderen Ausführungsform der Erfindung wird der Interrogations-Primer so ausgewählt, dass er *n* Nukleotidreste entfernt von dem nachzuweisenden SNP an ein Sondenmolekül hybridisiert. Dabei ist bezüglich der Anzahl *n* von Nukleotidresten zwischen dem 3'-Ende des Primers und dem variablen Nukleotid lediglich zu beachten, dass innerhalb der *n* Nukleotidreste kein Nukleotidrest vorkommt, der mit dem nachzuweisenden Nukleotid identisch ist.

Erfindungsgemäß kann der Nachweis mit unterschiedlich vielen markierten Nukleotiden bzw. Nukleotidanaloga erfolgen. Üblicherweise werden als Markierungen Fluoreszenzmarker verwendet (siehe unten), wobei in der Regel entweder 2 oder 4 markierte Nukleotide bzw. Analoga eingesetzt werden. Entsprechend spricht man vom *2-Color-* und *4-Color Approach.*

Alternativ zu dieser Versuchsabfolge werden auch andere Primer-Extensionsprotokolle angewandt. Eine sehr wichtige Methode stellt die Allel-spezifische Primer-Extension dar. Der wichtigste Unterschied zu dem bereits dargestellten Verfahren ist in den verwendeten Sonden zu sehen. Die 5'-aminomodifizierten Sondenmoleküle werden so entworfen, dass sie nicht ein Nukleotid 3' vor der polymorphen Position enden, sondern sie liegen mit ihrem letzten 3'-Nukleotid genau auf der polymorphen Position. Zu diesem Zweck werden pro zu analysierendem Locus 4 Sonden identischer Sequenz benötigt, die sich lediglich im letzten 3'-Nukleotid (dem SNP-Nukleotid) unterscheiden. Nach Hybridisierung mit dem Probenmolekül (in 4 individuellen Reaktionsgefäßen) und dem Aufspotten, wird bei der Extensionsreaktion nur einer dieser 4 Extensionsprimer verlängert, nämlich der mit dem komplementären SNP-Nukleotid an seinem 3'-Ende. Die anderen 3 Extensionsprimer werden dadurch, dass das letzte 3'-Nukleotid mit dem Probenmolekül nicht paaren kann, auch nicht verlängert (Extension bleibt aus). Zum Nachweis, welcher Extensionsprimer verlängert wurde, werden hier mit ein und dem selben Fluorochrom markierte dNTPs (Nukleotide A, C, G und T) verwendet. Die Identität des SNP-Nukleotids wird dadurch ermittelt, welcher der 4 Extensionsprimer ein Fluoreszenzsignal emittiert, also das korrekte Nukleotid in der letzten 3'-Position trägt.

Weiterhin könnten die eingesetzten einzelsträngig synthetisierten Extensionsprimer selbst Hybridnatur in dem Sinne aufweisen, dass sie nur in ihrem 3'-Bereich der Probe komplementär sind, in ihrem 5'-Bereich jedoch zusätzliche Sequenzen besitzen (nochmals ca. 20 bp), die nur zu den verwendeten PCR-Primern komplementär sind (künstlich komplementäre Sequenzen). Dadurch kann ein sog. Adressensystem aufgebaut werden, welches erstens die Hybridisierung unterstützt und die Nukleotidpaarungen verstärkt (durch die erhöhte Menge an Wasserstoffbrücken), und zweitens im Falle von partieller oder totaler Denaturierung des Hybridstrangs dazu führt, dass sich Probe im Reaktionsgemisch und Sonde auf dem Chip unter Umständen trotzdem wiederfinden könnten. Für diese Aufgabe sind auch Nukleotidanalogons vorstellbar.

Nicht verwechselt werden sollten die genannten Nachweismethoden mit der Allelspezifischen Hybridisierung, bei der keine Extension einer Nukleinsäure, sondern lediglich Hybridisierung von Sondenoligonukleotiden und markierter Probe in der Hybridisierungslösung zum Nachweis durchgeführt werden. Die Spezifizität der Hybridisierung wird in diesem Fall über so genannte *Mismatch-*Oligonukleotide geregelt (WO89/10977; Southern et al. (1992), Genomics, 13, 1008-1017). Dabei sind bis zu 20 Oligonukleotide pro zu analysierendem Locus notwendig (wegen Permutation der *Mismatch*-Positionen). Dieses Nachweisverfahren eignet sich ebenfalls für das erfindungsgemäße Verfahren.

Auch der Nachweis von Insertionen oder Deletionen kann durch Primer-Extensionsanalyse erfolgen. Dabei ist im Falle der Deletion zu beachten, dass die erste Base in der Deletion nicht mit der ersten Basen nach der Deletion identisch ist. Beim Nachweis von Insertionen ist zu beachten, dass die erste Base der Insertion nicht mit der ersten Base nach der Insertion identisch ist.

Erfindungsgemäß bedeutet der Begriff "Hybridisierung" oder "hybridisieren", dass zwei Stränge von Nukleinsäuremolekülen in einer Sequenz-abhängigen Weise Wasserstoffbrücken ausbilden. Zum Beispiel können unter geeigneten Bedingungen, die dem Fachmann bekannt sind, komplementäre Nukleotidsequenzen miteinander hybridisieren, um doppelsträngige DNA oder RNA zu bilden, oder ein doppelsträngiges Hybrid aus RNA und DNA. Siehe im Zusammenhang mit Hybridisierung auch Sambrook et al., *vide supra;* Ausubel et al., Current Protocols in Molecular Biology, Green Publishing Associates and Wiley Interscience, New York (1989); oder Higgins and Harnes, Nucleic Acid Hybridization, A Practical Approach, EAL Press Oxford, Washington D.C. (1985), die hier ausdrücklich als Referenz beigefügt sind.

Erfindungsgemäß wird unter Hybridisierung die Bildung von doppelsträngigen Nukleinsäuremolekülen aus komplementären einzelsträngigen Nukleinsäuremolekülen verstanden, wobei sich die Komplementarität aus den Basensequenzen der einzelsträngigen Moleküle ergibt. Bei den doppelsträngigen Nukleinsäuremolekülen kann es sich z.B. um DNA-DNA, DNA-RNA oder RNA-RNA-Duplexmoleküle handeln. Hybridisierungsexperimente werden üblicherweise eingesetzt, um Komplementarität zwischen verschiedenen einzelsträngigen Nukleinsäuremolekülen nachzuweisen. Die Bildung eines stabilen doppelsträngigen Moleküls hängt dabei von der Stringenz der Reaktionsbedingungen ab.

Als stringente Bedingungen werden Reaktionsbedingungen bei Nukleinsäurehybridisierungen bezeichnet, die die spezifische Bildung einer doppelsträngigen Duplex erlauben, d.h. aus einzelsträngigen Nukleinsäuremolekülen mit einer 100%igen oder einem hohen Anteil an Basenkomplementarität erlauben.

Unter einem hohen Anteil an Basenkomplementarität wird dabei erfindungsgemäß eine Basenkomplementarität der miteinander zu hybridisierenden einzelsträngigen Nukleinsäuremoleküle von mindestens 60%, bevorzugt von mindestens 70%, ebenfalls besonders bevorzugt von mindestens 80%, insbesondere bevorzugt von mindestens 90% und am meisten bevorzugt von mindestens 95 oder 98% verstanden.

Dem Fachmann ist geläufig, dass die Stringenz der Reaktion von den Temperaturen des Hybridisierungsschritts und der Waschschritte sowie von den Salzkonzentrationen und pH-Werten der Hybridisierungs- und Waschpuffer abhängt. Eine Erhöhung der Stringenz kann dabei durch die Verwendung von zunehmend verdünnten Salzlösungen erreicht werden. Darüber hinaus können stringentere Reaktionsbedingungen durch Zugabe von die Ausbildung einer Duplex destabilisierenden Agenzien wie z.B. Formamid erreicht werden.

Dem Fachmann ist bekannt, welchen Einfluss die Änderung der Temperatur der Hybridisierungs- und Waschschritte, der Salzkonzentration, des pH-Werts und der sonstigen Zusammensetzung der Wasch- und Hybridisierungspuffer auf die Stringenz der Reaktionsbedingungen haben. Im einzelnen können diese z.B. in Sambrook et al. (2001) *vide supra* nachgelesen werden.

Typische stringente Hybridisierungsbedingungen umfassen eine Hybridisierungs- und Waschtemperatur von 50°C bis 85°C. Typische Hybridisierungspuffer für diese Bedingungen sind 5x SSC (Sambrook et al., *vide supra*)*,* typische Waschpuffer sind 1x SSC mit 0,1% SDS (Sambrook et al., *vide supra*).

Erfindungsgemäß werden als niedrig-stringente Bedingungen solche Reaktionsbedingungen bezeichnet, bei denen im Verlauf einer Nukleinsäure-Hybridisierung die Bildung einer doppelsträngigen Duplex aus einzelsträngigen Nukleinsäuremolekülen mit einer eingeschränkten Basenkomplementarität ermöglicht wird.

Nukleinsäuremoleküle weisen erfindungsgemäß eine eingeschränkte Basenkomplementarität auf, wenn sie zu mindestens 30%, bevorzugt zu mindestens 40%, und besonders bevorzugt zu mindestens 50% zueinander komplementär sind. Dem Fachmann ist bekannt, wie er die Temperaturen und Pufferbedingungen während der Hybridisierungs- und Waschschritte im Rahmen einer Hybridisierungsreaktion wählen muss, um niedrig-stringente Reaktionsbedingungen einzustellen.

Als "annealing" wird definitionsgemäß der Vorgang bezeichnet, bei dem sich zwei einzelsträngige Nukleinsäuremoleküle aufgrund ihrer Basenkomplementarität aneinander einlagern, miteinander wechselwirken und doppelsträngige Strukturen bzw. eine doppelsträngige Helix bzw. eine Duplex ausbilden. Die Wechselwirkung zwischen den einzelsträngigen Nukleinsäuremolekülen basiert auf der Wasserstoffbrückenbindung zwischen komplementären Basenpaaren in den einzelnen Strängen. Auf diese Weise können doppelsträngige DNA-DNA-Helices, DNA-RNA-Helices oder RNA-RNA-Helices ausgebildet werden.

Als Hybrid wird im Rahmen der vorliegenden Erfindung ein doppelsträngiges Nukleinsäuremolekül bezeichnet, dessen Einzelstränge aus verschiedenen Nukleinsäuremolekülen stammen und im Rahmen einer Hybridisierung, d.h. einer aufeinanderfolgenden Denaturierung und Renaturierung entstanden sind.

Die bei einer bevorzugten Ausführung durch die 5'-Exonukleasebehandlung einzelsträngig in MTPs (Mikrotiterplatten) vorliegenden individuellen Probenmoleküle werden mit den passenden einzelsträngig synthetisierten Sondenmolekülen in Standardhybridisierungslösungen gemischt. Solche Lösungen enthalten z.B. 5x SSC.

Die MTP wird auf ca. 85°C erhitzt, dann langsam auf 40°C abgekühlt und auf Eis gestellt. Probe und Sonde können aber auch bei einer bestimmten, vorher aus der Sequenz von Probe und Sonde errechneten, Temperatur inkubiert werden, um so die Hybridisierung herbeizuführen. Ist die korrekte Temperatur, die so genannte Tm, (z.B. während des Kühlens) erreicht, lagert sich das Sondenmolekül an den Probeneinzelstrang an (Hybridisierung). Der Prozess des langsamen Kühlens stellt sicher, dass jedes Temperaturoptimum für jede individuelle Hybridisierung in der MTP erreicht wird.

Durch die Positionierung der Sonde während der Hybridisierung am 3'-Ende des genomischen Einzelstrangs (Probe) ist Komplementarität gewährleistet. Durch die Sondenhybridisierung am 3'-Ende der Einzelstrangprobe ist sichergestellt, dass der *Spacer* mit seinem *Linker* über das Ende des Hybridmoleküls aus Sonde und Probe hinausragt und so freier Zugang des *Linkers* zur Chipoberfläche besteht.

Bevor diese Hybride auf den Chip aufgebracht werden, werden sie noch von nicht hybridisierten, also einzelsträngigen Molekülen getrennt und so ausschließlich verlängerbare Hybridmoleküle auf den Chip aufgebracht und dort kovalent gebunden. Dazu stehen mehrere Möglichkeiten zur Verfügung, wie z.B. selektive Ethanolfällung, Reinigungskits (von z.B. Biorad, Deutschland oder Bio110) oder Microconfilter mit entsprechenden Porengrößen (Millipore, Deutschland).

Z.B. kann die Abtrennung der doppelsträngigen Hybride durch selektive Ethanolfällung erfolgen. Dazu werden 2 Volumen 100% Ethanol zum Hybridisierungsansatz gegeben, das Präzipitat durch Zentrifugieren pelletiert und die Pellets mit 80%igem Ethanol gewaschen. Dadurch werden die nicht-hybridisierten, noch in Lösung befindlichen Einzelstrangproben und Oligonukleotid-Sonden entfernt. Die präzipitierten Hybride werden dann in Spottingpuffer aufgenommen.

Die Immobilisierung von Oligonukleotiden (synthetischen Sondenmolekülen) auf Biochips ist weitgehend standardisiert. Die kostengünstigste Alternative sind aminomodifizierte Sonden, die mit der Epoxysilan- (3-Glycidoxypropyltrimethoxysilan-) beschichteten Oberfläche von fluoreszenzfreien Mikroskop-Objektträgern reagieren. Im Handel sind noch weitere Beschichtungen und dazugehörige Modifikationen der Sonden erhältlich. Diese Beschichtungen umfassen z.B. Aldehyd-, Aminosilane- (3-Aminopropyltrimethoxysilane), Polylysin- oder Isothiocyanat-Beschichtungen oder Beschichtungen mit langkettigen, hydrophilen Polymeren, die amin-reaktive Gruppen enthalten. Die genannten Substrate reagieren bevorzugt mit Aminogruppen am Ende der Spacer von Sondenmolekülen. Eine weitere Alternative wäre eine Hybridbindung über das Biotin-Streptavidin-System. Dem Fachmann ist klar, dass die funktionellen Gruppen der Beschichtungen und des Linkers auch entsprechend vertauscht werden können. Weitere Immobilisierungsmöglichkeiten bestehen durch die Reaktion von Oligonukleotiden mit 5'-Disulfidgruppen mit mercaptosilan-derivatisierten Oberflächen sowie acrylamid-modifizierten Oligonukleotiden mit EZ-RAYS^{™}-Objektträgern. Werden unmodifizierte Objektträger verwendet, können silanisierte Oligonukleotide immobilisiert werden (Übersicht in Lindroos et al., (2001), Nucl. Acids Res., Bd. 29, Nr. 13, e69).

Bei einer bevorzugten Ausführung werden die MTP, in denen die Hybride in Spottingpuffer resuspendiert sind, in den Microarray-Roboter gegeben und die individuellen Hybridmoleküle mit adäquatem *Spacer* und *Linker* auf den Chip mit aktivierter Oberfläche aufgebracht. Die chemische Verbindung von Aminogruppen und Hybridmolekül und Epoxygruppen auf dem Chip erfolgt durch einfaches UV-*Crosslinking.* Alternativ können die Chips auch in einer feuchten Kammer inkubiert oder heiß gebacken werden. Hier ist anzumerken, dass nicht nur Mikroskop-Objektträger aus Glas oberflächenbeschichtet werden können, sondern auch andere Materialien, vornehmlich Plastik oder Keramik, eingesetzt werden. Typischerweise sind die Materialien optisch transparent oder verspiegelt.

Im nächsten Schritt findet bei einer bevorzugten Ausführung die bereits erwähnte Primer-Extension, also die enzymatische Verlängerung des Primers, der bevorzugt das Sondenmolekül darstellt, in Template-abhängiger Weise statt. Dabei wird die Primer-Extension allgemein so durchgeführt, wie sie im Stand der Technik beschrieben ist, wobei im Handel erhältliche Enzyme, Reagenzien und Kits eingesetzt werden können.

Allgemein wird das immobilisierte Hybrid aus Sonde und Probe mit einem oder mehreren Nukleosidtriphosphaten, einschließlich mindestens einem markierten oder modifizierten Nukleosidtriphosphat, zusammen mit einem Polymerisierungsmittel unter Bedingungen in Kontakt gebracht, die die Extension des Primers begünstigen. Hier können entweder markierte Desoxyribonukleosidtriphosphate (dNTPs) oder markierte kettenabbrechende Didesoxyribonukleosidtriphosphate (ddNTPs) eingesetzt werden. Das Polymerisierungsmittel wird den Primer mit dem zu dem variablen Nukleotid neben dem Primer komplementären Nukleosidtriphosphat verlängern.

Der Begriff "markiertes Nukleosidtriphosphat" schließt jedes Nukleosidtriphosphat, Desoxy- oder Didesoxynukleosidtriphosphat ein, das mit einem nachweisbaren Label oder Marker gelabelt bzw. markiert ist oder das derart modifiziert ist, dass es eine Gruppe oder einen Rest umfasst, die bzw. der in der Lage ist, einen detektierbaren Marker zu binden. Dabei spielt es im Rahmen der Erfindung keine Rolle, welcher Marker bzw. welches Label für den Nachweis eingesetzt wird. Allerdings unterscheiden sich verschiedene Marker natürlich hinsichtlich ihrer Handhabbarkeit, ihrer Kosten und natürlich auch ihrer Sensitivität. Wichtig ist allerdings, dass der nachweisbare Marker den Einbau des markierten Nukleosidtriphosphats während der Polymerisationsreaktion, die zur Verlängerung des Primers führt, nicht behindert oder fehlerhaft macht.

Als Fluoreszenzmarkierung der ddNTPs oder dNTPs werden hauptsächlich Cyanin-(z.B. Cy3 oder Cy5), Renaissance- (z.B. ROX oder R110) oder Fluorescein-Farbstoffe (z.B. FAM oder FITC) verwendet, also z.B. Cyanine-5-ddATP oder Renaissance-110-ddGTP oder entsprechende dNTPs. Weitere zur Fluoreszenz anregbare Farbstoffe werden ständig entwickelt und sind dem Fachmann bekannt.

Als alternative Markierungen können radioaktive Markierungen wie P³², das Biotin-Streptavidin-System oder Antigen-Antikörper-Systeme, die an Enzyme wie z.B. Meerrettich-Peroxidase gekoppelt sind, verwendet werden.

Der Begriff "Polymerisationsmittel" steht für jedes Enzym, das in der Lage ist, Nukleinsäuren in Template-abhängiger Weise zu verlängern. Geeignete Enzyme schließen z.B. ein Sequenase, T7 DNA-Polymerase, T4 DNA-Polymerase, das Klenow-Fragment der DNA-Polymerase aus *Escherichia coli* und andere geeignete DNA-Polymerasen, Reverse Transkriptase und Polymerasen aus thermophilen Mikroorganismen wie *Thermos aquaticus* und *Thermos thermophilus.*

In einer bevorzugten Ausführungsform der Erfindung handelt es sich bei dem Polymerisierungsmittel um Sequenase^{™}.

Die Hybride für die Template-abhängige Primer-Extension liegen fest gebunden im Microarray-Format auf der Chipoberfläche in trockenem Zustand vor und sind so auch lagerbar. Die Durchführung der multiparallelen Primer-Extension, bei der Tausende von Analysen in z.B. 50µl Volumen auf dem Chip durchgeführt werden können, stellt einen effektiven Rationalisierungs- und Kostensenkungsprozess dar. Würde die Extension in der MTP vorgenommen, müssten weitaus größere Mengen teuerer Reagenzien wie Sequenase^{™} und fluoreszenzmarkierte ddNTPs verbraucht werden.

Bei einer bevorzugten Ausführungsform beginnt die Extension auf dem Biochip mit dem in Kontakt bringen von Microarray und Extensionslösung in einer verdunstungssicheren Kammer auf dem Chip. Das Reaktionsgemisch, das z.B. 0,2 Units Sequenase^{™} pro µl und 6-8 µM fluoreszenzmarkierte ddNTPs in einem mit der Sequenase^{™} gelieferten Reaktionspuffer enthält, und der Microarray werden ca. 1h, bei unter diesen Umständen optimaler Temperatur für das verwendete Polymerisationsmittel, inkubiert und so die Primer-Extension durchgeführt.

Die Extensionslösung kann alle vier ddNTPs oder Analoga mit vier verschiedenen Farbstoffen markiert enthalten, oder aber nur zwei mit unterschiedlichen Farbstoffen markierte ddNTPs, da SNPs oft binäre Marker darstellen und bei vielen Anwendungen nur festgestellt werden soll, welcher der beiden möglichen Nukleotidzustände vorliegt. Da die markierten ddNTPs durch das Polymerisationsmittel fest an die Extensionsprimer (Sondenmolekül) angefügt werden und die Sonde fest mit der Unterlage (dem Chip) verbunden ist, ist das angefügte ddNTP ebenfalls irreversibel an den Chip gebunden.

Dies eröffnet die Möglichkeit einer stringenten Waschung des Chips z.B. mit H₂O, (Bidest, 80°C, 10min.), um Hintergrundrauschen zu eliminieren und nicht eingebaute ddNTPs zu entfernen. Hierdurch ist die spezifische Verlängerung der Sondenmoleküle im Probenpunkt eindeutig festzustellen, da die Wellenlänge (Farbe) der Fluoreszenz verrät, um welches ddNTP die Probe verlängert wurde. Die Probenpunkte haben typischerweise einen Durchmesser von ca. 80 bis 160µm.

Bei einer bevorzugten Ausführungsform hat das eingesetzte laserbasierte Anregungs-/Detektionssystem, welches die Fluorochrome analysiert, typischerweise eine Auflösung von 2µm und eine Sensitivität von 0,5 Fluorochromen pro µm². Meist werden zwei (oder vier) Fluorochromtypen gewählt, deren Anregungs- bzw. Emissionswellenlängen möglichst weit auseinander liegen. So wird z.B. Cy5 bei 650 nm angeregt und emittiert bei 667 nm (Filter 675 nm ±10), R110 wird mit einem 488nm Laser angeregt und emittiert bei 525 nm (Filter 512nm ±15). Dies stellt sicher, dass sich die Signale der beiden Farbstoffe nicht überlagern, da sie durch die entsprechenden Filtersysteme eindeutig voneinander trennbar sind. Das mit dem Scanner kompatible Analyseprogramm erkennt Wellenlänge (Farbe) und Intensitität jedes Probenpunktes und legt die Ergebnisse direkt in einer Datenbank ab.

Wie bereits ausgeführt, können beim erfindungsgemäßen Verfahren mehrere genomische Loci mehrerer Individuen gleichzeitig nachgewiesen werden.

Dazu werden üblicherweise mehrere Sonden, die jeweils zum Nachweis unterschiedlicher genomischer Loci verwendet werden können, zusammen mit dem jeweils hybridisierten Probenmolekül auf dem Träger des Chips im Arrayformat abgelegt. Dabei werden mindestens 1 bis 20000 unterschiedliche Sonden, mindestens 48 bis 15360 unterschiedliche Sonden, bevorzugt mindestens 96 bis 15360 unterschiedliche Sonden, ebenfalls besonders bevorzugt mindestens 96 bis 6144 unterschiedliche Sonden, insbesondere bevorzugt mindestens 96 bis 1536 unterschiedliche Sonden und am meisten bevorzugt mindestens 96 bis 384 unterschiedliche Sonden auf dem Träger untersucht.

Der Fachmann weiß, dass die maximale Anzahl an Sonden, die auf einem Microarray aufgebracht werden kann, durch die Synthesemethode des Array vorgegeben ist. Die angegebenen Zahlen resultieren darüber hinaus daraus, dass die typischen MTPs, in denen die Erfindung zu Zwecken der Automatisierung bevorzugt durchgeführt wird, 96 Vertiefungen bzw. ganzzahlige Vielfache davon aufweisen.

Erfindungsgemäß können je nach Nachweismethode die einzelnen unterschiedlichen Sonden auch mehrfach auf dem Träger abgelegt sein. Bevorzugt wird eine einzelne Sonde mindestens 10 Mal, ebenfalls bevorzugt mindestens 5 Mal, besonders bevorzugt mindestens 4, 3 oder 2 Mal auf dem Träger abgelegt.

Erfindungsgemäß weisen die Sondenmoleküle eine Länge von mindestens 10 bis 100 Nukleotiden, bevorzugt von mindestens 20 bis 75 Nukleotiden, besonders bevorzugt von mindestens 20 bis 50 Nukleotiden, insbesondere bevorzugt von mindestens 20 bis 40 Nukleotiden, ebenfalls insbesondere von 20 bis 35 Nukleotiden und am meisten bevorzugt von mindestens 20 bis 30 Nukleotiden auf.

Erfindungsgemäß weisen die Probenmoleküle eine Länge von mindestens 30 bis 250 Nukleotiden, von mindestens 30 bis 200 Nukleotiden, bevorzugt von mindestens 40 bis 160 Nukleotiden, besonders bevorzugt von mindestens 40 bis 120 Nukleotiden, insbesondere bevorzugt von mindestens 50 bis 100 Nukleotiden und am meisten bevorzugt von etwa 80 Nukleotiden auf.

Bevorzugt werden bei dem erfindungsgemäßen Verfahren mindestens 1 bis 20000 unterschiedliche Individuen, mindestens 48 bis 15360 unterschiedliche Individuen, bevorzugt mindestens 96 bis 15360 unterschiedliche Individuen, besonders bevorzugt mindestens 96 bis 6144 unterschiedliche Individuen, insbesondere bevorzugt mindestens 96 bis 1536 unterschiedliche Individuen und am meisten bevorzugt mindestens 96 bis 384 Individuen auf dem Träger untersucht.

Erfindungsgemäß werden bei dem Verfahren mindestens 1 bis 20000 unterschiedliche Individuen, mindestens 48 bis 15360 unterschiedliche Individuen, bevorzugt mindestens 96 bis 15360 unterschiedliche Individuen, besonders bevorzugt mindestens 96 bis 6144 unterschiedliche Individuen, insbesondere bevorzugt mindestens 96 bis 1536 unterschiedliche Individuen bezüglich mindestens 1 bis 20000 unterschiedlicher Loci, mindestens 48 bis 15360 unterschiedlicher Loci, bevorzugt mindestens 96 bis 15360 unterschiedlicher Loci, besonders bevorzugt mindestens 96 bis 6144 unterschiedlicher Loci, insbesondere bevorzugt mindestens 96 bis 1536 unterschiedlicher Loci und am meisten bevorzugt mindestens 96 bis 384 unterschiedlicher Loci parallel untersucht.

Erfindungsgemäß können verschiedene Individuen verschiedener Organismen hinsichtlich genetischer Polymorphismen wie SNPs untersucht werden. Dazu gehören z.B. Mikroorganismen, bevorzugt *E.coli* und/oder human-pathogene Mikroorganismen, Pilze, bevorzugt *Saccharomyces cerevisiae, Schizosaccharomyces pombe* und/oder Pflanzen-pathogene Pilze, Ratte, Rind, Maus, Mensch oder Pflanzen.

Bei den Pflanzen werden bevorzugt Individuen von monokotylen oder dikotylen Nutz-, Zier-, Nahrungs- oder Futterpflanzen durch das erfindungsgemäße Verfahren charakterisiert. Beispiele für monokotyle Pflanzen sind Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören. Dikotyle Nutzpflanzen umfassen unter anderem Baumwolle, Leguminosen wie Hülsenfrüchte und insbesondere Alfalfa, Sojabohne, Raps, Tomate, Zuckerrübe, Kartoffel, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitalis umfassen. Besonders bevorzugt sind die Getreide Weizen, Roggen, Hafer, Gerste, Reis, Mais und Hirse, sowie Zuckerrübe, Raps, Soja, Tomate, Kartoffel und Tabak. Weitere Nutzpflanzen können dem US-Patent US 6,137,030 entnommen werden.

Besonders bevorzugt werden erfindungsgemäß Gerste, *Arabidopsis,* Raps und Mais untersucht.

Bevorzugt werden durch das erfindungsgemäße Verfahren 96 Loci von 6144 Individuen Gerste, *Arabidopsis,* Raps oder Mais analysiert.

Das kennzeichnende Merkmal des erfindungsgemäßen Verfahrens, das zeitliche Vorziehen eines Verfahrensschritts, der dazu führt, dass Probe und Sonde bereits vor der Positionierung auf dem Chip zueinander finden, ist natürlich auch für Nukleinsäureanaloga wie etwa PNAs durchführbar. Auch können Probe und Sonde ganz anderer Natur sein, z.B. Proteine oder Peptide im Antikörperscreening oder auch Kohlenhydrate wie Zuckerketten an Glycoproteinen zum Screening von niedermolekularen Liganden (Antigenen) aller Stoffklassen.

Das erfindungsgemäße Verfahren kann somit auf alle Nachweisverfahren angewendet werden, bei denen parallel die spezifische Interaktion zwischen zwei Molekülen (Sonde und Probe), die zueinander komplementäre Bindungseigenschaften haben, durch Hybridbildung nachgewiesen wird.

Insofern ist das erfindungsgemäße Verfahren nicht auf den Nachweis von SNPs beschränkt, sondern kann auch verwendet werden, um parallel mehrere Spezies in einer Probe, z.B. durch rDNA-Hybridisierung nachzuweisen. Ebenfalls kann die Genaktivität einer Zelle durch das erfindungsgemäße Verfahren nachgewiesen werden.

Dabei ist dem Fachmann bekannt, dass der Nachweis der Hybride auf dem Chip durch eine Reihe von zusätzlichen, bisher nicht erwähnten Nachweismethoden erfolgen kann. Beim rDNA-basierten Nachweis von Mikroorganismen können die Probenmoleküle z.B. durch Verwendung entsprechend markierter Primer bereits während der Amplifikation z.B. mit den bereits erwähnten Fluorophoren markiert werden. Das Nachweisverfahren besteht darin festzustellen, ob markierte Hybride auf dem Mikroarray nachgewiesen werden können. Das gleiche Nachweisverfahren kann bei der Analyse der Genaktivität angewandt werden, wenn während der PCR-Amplifikation der cDNA (siehe oben) entprechend markierte Primer eingesetzt werden. Weitere Nachweismethoden sind dem Fachmann bekannt.

Gegenstand der Erfindung sind ebenfalls die im Rahmen der Beispiele dargestellten Primer, Sonden- und Probenmoleküle.

Gegenstand der Erfindung ist auch die Verwendung der in den Beispielen dargestellten Primer, Sonden- und Probenmoleküle in einem der erfindungsgemäßen Verfahren.

Ein erfindungsgemäßes Verfahren kann üblicherweise die folgenden Schritte umfassen:
Im Folgenden wird zunächst ein Beispiel angegeben, das in exemplarischer Weise verdeutlicht, wie durch ein erfindungsgemäßes Verfahren mehrere SNPs mehrerer Individuen nachgewiesen werden können. Dem Fachmann ist dabei geläufig, wie er durch Optimierung und Variation der einzelnen dargestellten experimentellen Schritte das Verfahren seinen jeweiligen Bedürfnissen anpassen kann. Die Beispiele sind nicht einschränkend zu deuten.

Zunächst werden singuläre SNPs mittels Bioinformatik und öffentlichen Sequenzdatenbanken ermittelt. Durch unterschiedliche Genom-Projekte stehen Millionen von Sequenzen exprimierter Gene (cDNAs), aber auch Gesamtgenome zur Verfügung. Darüber hinaus gibt es Datenbanken (z.B. am NCBI, http://www.ncbi.nlm.nih.gov), die explizit die Position von SNPs angeben. Ausgehend von diesen SNP-Datenbanken oder durch Vergleich von eigenen Sequenzdaten mit den entsprechenden Referenzsequenzen in öffentlich zugänglichen Datenbanken können SNPs identifiziert werden.

In einem zweiten Schritt werden dann Primer für eine PCR abgeleitet, die das genomische Fragment amplifizieren, das den nachzuweisenden Polymorphismus trägt. Dabei muss ein Teilbereich eines der beiden Primer zu einem Teilbereich des späteren Extensionsprimers (der Sonde) komplementär sein, um die Hybridisierung der Sonde mit dem Target mit 100%iger Sicherheit zu garantieren. Der Primer, der zur Amplifikation des zur Hybridisierung benötigten Strangs verwendet wird, kann eine Modifikation tragen, die davor schützt, von einer 5'-Exonuklease abgebaut zu werden (z.B. eine Phosphoro-Thioat-Modifikation). Primer können z.B. mit der Software Primer3 (Whitehead Institute, MIT, Steve Rozen, Helen j. Skaletsky, 1996 und 1997; abrufbar unter http://www.genome.wi.mit.edu/genome_software/other/primer3.html) abgeleitet werden.

Optional kann eine vergleichende Sequenzierung der amplifizierten Fragmente in verschiedenen genetischen Hintergründen zur Verifikation, Qualitätskontrolle und Auswahl geeigneter Marker durchgeführt werden.

Im nächsten Schritt werden dann 5'-amino-modifizierte Sondenmoleküle ebenfalls mit der Primer3-Software entworfen und synthetisiert. Die Bestellung erfolgt bei bekannten Herstellern wie Metabion, Martinsried, Deutschland. Die Sonden sind so gewählt, dass sie an ihrem 3'-Ende genau ein Nukleotid vor der polymorphen Position des zu untersuchenden Polymorphismus enden.

Unter Verwendung der bereits erwähnten PCR-Primer werden dann die verschiedenen genomischen Loci der verschiedenen zu untersuchenden Individuen durch PCR amplifiziert. Die Annealingtemperatur der Primer liegt zwischen 50 und 68°C, bevorzugt zwischen 55 und 60°C. Die Größe der amplifizierten Fragmente liegt zwischen 40 bis 200 Basenpaaren, bevorzugt zwischen 50 und 150 Basenpaaren, besonders bevorzugt zwischen 50 und 100 Basenpaaren, insbesondere zwischen 50 und 80 Basenpaaren und am meisten bevorzugt zwischen 50 und 70 Basenpaaren.

Typische Reaktionsbedingungen umfassen:
10 ng/µl genom DNA Template
0,2 mM dNTPs
1,5 mM MgCl₂
10x Reaktionspuffer
je 5 µM forward und reverse Primer
0,02 U/µl Taq-Polymerase

Die Polymerase und der Reaktionspuffer werden üblicherweise von Qiagen, Deutschland bezogen.

Das Temperaturprotokoll der PCR sieht üblicherweise folgendermaßen aus:

| | | | |
|---|---|---|---|
| 1 Zyklus: | 5 min | bei | 94°C |
| 35 Zyklen: | 20 sec | bei | 94°C |
| | 30 sec | bei | Annealingtemperatur (50-68°C) |
| | 25 sec | bei | 72°C |
| 1 Zyklus | 2 min | bei | 72°C |

Anschließend erfolgt der Verdau des nicht geschützten amplifizierten Strangs durch eine hinsichtlich der PTO-Modifikation selektive Exonuklease, z. B. T7 Gen 6 5'-Endonuklease, nach den Herstellerangaben (Amersham-Pharmacia Biotech, Schweden).

Nach Inaktivierung der 5'-Exonuklease (z.B. Inkubation für 5 Minuten bei 90°C), wird dann die Einzelstrangprobe mit dem Sonden-Oligonukleotid in einem Standardhybridisierungspuffer (6x SSC nach Sambrook et al., *vide supra*) auf Mikrotiterplatten gemischt. Die Positionen der Sonde und untersuchten Individuen sind bekannt.

Die Hybridisierung zwischen Einzelstrangprobe und der Oligonukleotid-Sonde erfolgt üblicherweise durch Erhitzen auf 85° C und langsames Abkühlen der Hybridisierungslösung mit 0,5°C/min. Dadurch wird ein Hybrid aus Probe und Sonde hergestellt.

Die Aufarbeitung der Hybride und Abtrennung von nicht-hybridisierten Einzelstrangmolekülen erfolgt durch selektive Ethanolfällung. Dazu werden 2 Volumen 100% Ethanol zum Hybridisierungsansatz gegeben, das Präzipitat durch Zentrifugieren pelletiert und die Pellets mit 80%igem Ethanol gewaschen. Dadurch werden die nicht-hybridisierten, noch in Lösung befindlichen Einzelstrangproben und Oligonukleotid-Sonden entfernt.

Die präzipitierten doppelsträngigen Hybride werden dann in einem geeigneten Spottingpuffer wie der Link_solution der Quantifoil in Jena, Deutschland aufgenommen und auf Epoxy- oder z.B silanisierte, fluoreszenzfreie Mikroobjektträger mit Hilfe eines Microarray-Roboters, der die Bildung von Datenpunkten mit einem Durchmesser von 0,08 bis 0,16 mm erlaubt, gespottet.

Durch einen kurzen UV-Crosslink in einer gesättigten Dampfatmosphäre bei 50-60°C werden die Hybride kovalent an den Träger gekoppelt und bei 90°C eingebacken. Hiermit ist der Chip fertiggestellt.

Es folgt die Durchführung der Extensionsreaktion auf dem Chip, die mit Sequenase^{™} und unterschiedlich fluoreszenzmarkierten ddNTPs in Sequenase^{™}-Puffer bei 50-80°C durchgeführt wird.

Der SNP-Nachweis erfolgt in diesem Fall durch die Verlängeurng des Hybridmoleküls auf der Chipoberfläche um ein fluoreszenzmarkiertes Nukleotid (sog. single base extension), wobei das Sonden-Oligonukleotid als Primer und die hybridisierte Einzelstrangprobe als Template fungieren. Da die Nukleotide unterschiedlich fluoreszenzmarkiert sind und nicht weiter im Rahmen der Extensionsreaktion verlängert werden können, wird nur das Nukleotid eingebaut, das zu dem jeweiligen SNP komplementär ist.

Nach Durchführung der Extensionsreaktion wird der Chip mit heißem destilliertem Wasser, das mit bis zu 0,1-2% SDS versetzt sein kann, gewaschen und getrocknet. Dann wird der Chip in einen Laserscanner gegeben, der in wenigen Sekunden die Farbe (emittierte Wellenlänge) des verlängerten Sondenmoleküls erkennt und aufgrund der gemessenen Wellenlänge die Identität des SNPs am jeweiligen Probenpunkt auf dem Chip feststellt. Hierbei werden die physikalischen Eigenschaften der Farbstoffe bei der Wahl der Laser/Filter-Systeme nach Empfehlung der Hersteller (z.B. Genomic Solutions, USA) berücksichtigt. Die Ergebnisse werden als Intensitätswerte der Fluoreszenz (z.B. 65536 Graustufen bei den Wellenlängen der fluoreszenzmarkierten ddNTPs) direkt in einer Datenbank abgelegt.

Im folgenden wird die Erfindung an konkreten Beispielen erläutert. Diese sind nicht einschränkend zu deuten.

### Beispiel 1: Nachweis von 48 Loci in 2 Kultivaren

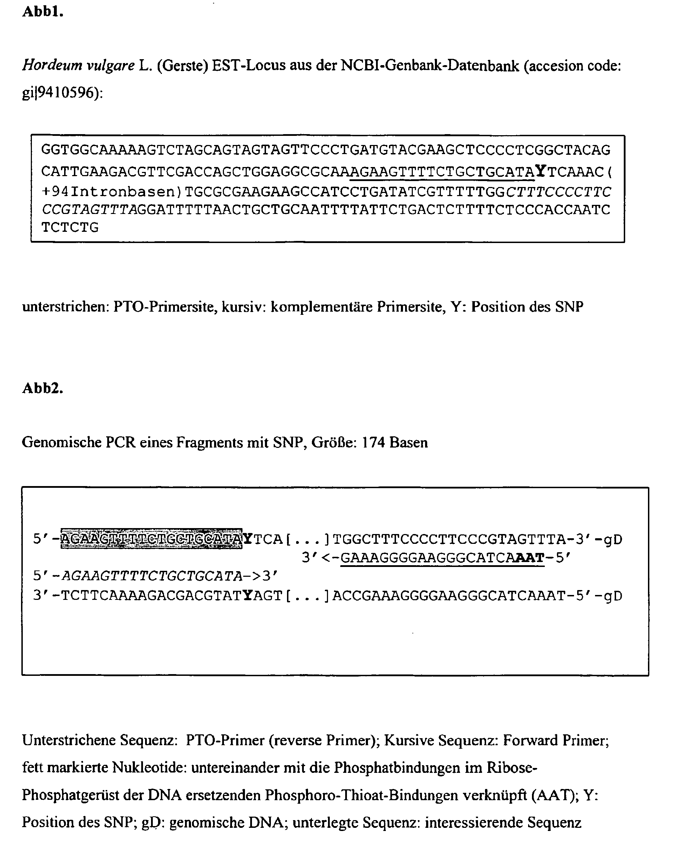

Im folgenden wird der Nachweis von 48 Loci in 2 Kultivaren der Gerste beschrieben. In Abb. 1 bis 3 sind beispielhaft die Position der zur PCR verwendeten Primer bezüglich eines der untersuchten Loci dargestellt. Im Beispiel werden SNPs bezüglich Thymidin (T) und Cytosin (C) untersucht.

Zunächst wurden die Sequenzinformationen von 48 singulären NukleotidPolymorphismen (SNPs) von *Hordeum vulgare* L mittels Bioinformatik in EST- und genomischen Sequenzdatenbanken beim NCBI (http://www.ncbi.nlm.nih.gov) ermittelt. Der Beispiel-Locus hat den Accession code: gi9410596.

Dann wurden genomische PCR-Primer mit der Software Primer3 (Whitehead Institute, Massachusetts Institute of Technology (MIT) Steve Rozen, Helen J. Skaletsky, 1996, 1997) entworfen. Die Kompilierung des Quellcodes unter SuSE Linux, Kernel 2.4. erfolgte nach Angaben des Programmierers (in der Dokumentation beim Quellcode und von der Internetseite http://www.genome.wi.mit.edu/genome_software/other/primer3.html).

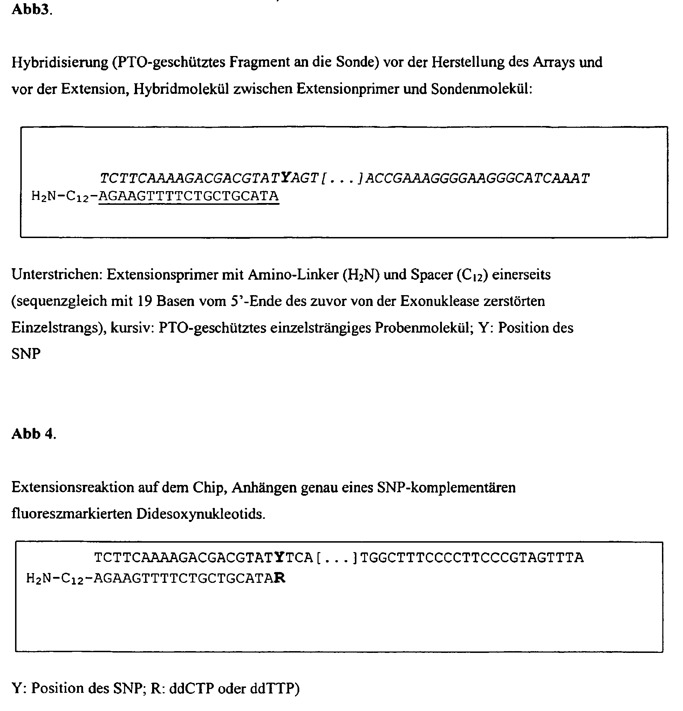

Die genomischen Primer wurden so entworfen, dass sie genomische Fragmente amplifizieren, die die zu untersuchenden SNPs tragen (Abbildung (Abb) 1, Y). Einer der beiden Primer, nämlich der geschützte PTO-Primer war zum späteren Extensionsprimer (Sonde, Abb. 3 und Abb. 4, kursives Oligo) komplementär, um die fehlerfreie Hybridisierung der Sonde vom 5'Ende her der dann einzelsträngigen Probe zu garantieren. Der Primer des hybridisierenden Halbstrangs trug eine Modifikation, die ihn davor schützte, von einer 5'-Exonuklease abgebaut zu werden. Dies war eine Phosphoro-Thioat-Modifikation (PTO) der Bindungen zwischen einer oder mehreren Basen vom 5'-Ende her.

Es wurde dann eine vergleichende Sequenzierung (ABI Sequenzer 3700, Rhodamin-Sequencing-Kit, Applied Biosystems, USA) der amplifizierten PCR-Fragmente mit jeweils einem der beiden PCR-Primer in verschiedenen genetischen Hintergründen zur Verifikation, Qualitätskontrolle und Auswahl geeigneter Marker durchgeführt.

Anschließend wurden 5'-aminomodifizierte Sondenmoleküle entworfen, die 3' genau ein Nukleotid vor der polymorphen Position enden (Software Primer3, siehe oben). Die Synthese der Sonden wurde bei der Metabion GmbH, Deutschland in Auftrag gegeben. Die Sondenmoleküle trugen als funktionelle Linker-Gruppe eine NH₂-Gruppe und waren über einen C₁₂-Spacer an die Sonde gekoppelt.

Danach wurden die 48 PCR-Fragmente aus genomischer DNA der 2 Kultivaren amplifiziert. Ein Beispiel für genomische Primer am Locus ist in Abb. 2 gegeben.

Im Beispiel betrug die Annealingtemperatur 51°C. Die Größe des amplifizierten Fragments betrug 174 Basenpaare. Die PCR wurde gemäß den Angaben des Polymeraseherstellers (Qiagen GmbH, Deutschland) nach folgendem Protokoll durchgeführt:

| | |
|---|---|
| 10 ng/µl | genomische DNA |
| 0,2 mM | dNTPs |
| 1,5 mM | MgCl₂ |
| 10x | Taq-Reaktionspuffer (nach Hersteller-Angaben) |
| je 5µM | forward und reverse PCR-Primer |
| 0,02 Units/µl | Taq-Polymerase |

Folgendes Temperatur-Protokoll wurde verwendet:

| | | | |
|---|---|---|---|
| 35 Zyklen: | 20 sec | bei | 94°C |
| | 30 sec | bei | 51°C |
| | 25 sec | bei | 72°C |

Danach wurden die amplifizierten PCR-Fragmente durch Zugabe von 2 Volumen 100% Ethanol bei 4°C und 1/10 Volumen 3 M Natriumacetat pH 4,5 gefällt, und bei 14000 rpm und 4°C für 45 min zentrifugiert. Anschließend wurde das Präzipitat durch Zugabe von 70% Ethanol gewaschen und erneut bei 14000 rpm und 4°C für 15 min zentrifugiert. Nach Trocknung wurde das Pellet in 6 µl destilliertem Wasser aufgenommen.

Danach erfolgte der Abbau des nicht mit den 5'PTO-Bindungen versehenen und daher ungeschützten (Gegen-)Strangs in den Fragmenten durch die 5'Exonuklease *T7* Gen 6 (Amersham Pharmacia-Biotech) unter Schonung des für die Analyse relevanten DNA-Strangs. Das Protokoll sah folgendermaßen aus:

| | |
|---|---|
| 2 µl | gefälltes PCR-Produkt |
| 0,1 µl | Exonuklease |
| 1,9 µl | dest. Wasser |
| 1,0 µl | 5 x Exonukleasepuffer (siehe Herstellerangaben) |

Inkubation für 60 min bei 37°C.

Danach erfolgte die Inaktivierung der 5'-Exonuklease durch Erhitzen für 20 min bei 85°C.

Anschließend wurde jeweils 1 µl 80 µM Sonde (Extensionsprimer) zur vom Gegenstrang befreiten Einzelstrangprobe gegeben.

Danach erfolgt eine Hybridisierungs-Inkubation der erhaltenen 6µl im Thermocycler, wobei über 45 min gleichmäßig von 85°C auf 40°C abgekühlt wurde.

Zur Abtrennung der nicht hybridisierten Einzelstränge und Nukleotide wurde der Hybridisierungsansatz bei Raumtemperatur mit 80µl (mindestens 2 Volumen) 100% Ethanol, 3µl 3M Natriumacetat pH 4,5 für 2 h bei -20°C präzipitiert. Nach 45 min Zentrifugation bei 14.000 rpm und 4°C, Waschen des Präzipitats mit 100µl 80% Ethanol, 15 min Zentrifugation bei 14.000 rpm und 4°C wurde das Pellet getrocknet und die präzipitierten doppelsträngigen Hybride in 3µl dest. Wasser mit 0,002% SDS und 0,2 % Polyethylenglykol 5000 aufgenommen.

Nach weiterer Zugabe von 3µl Spotting-Puffer (Link-Solution, Quantifoil GmbH, Deutschland) resultierten 6µl Lösung für das Aufbringen im Microarray. Vorteilhaft ist hier eine Endkonzentration von 40 µM des Extensionsprimers und damit des Hybridmoleküls in der Lösung.

Die Hybridmoleküle wurden auf kommerziell erhältliche Epoxy-modifizierte Objektträger (EpoxySlides, Quantifoil GmbH, Deutschland) mit Hilfe eines *Microarrayers* mit geschlitzten Nadeln (Microgrid II 600 TAS, Fa. Biorobotics Ltd. sowie zugehörige Steuersoftware und Dokumentation) aufgebracht. Die Datenpunkte hatten einen Durchmesser von ca. 150µm und Mittelpunkts-Distanzen von 250µm.

Danach erfolgte die Bindereaktion:
- 5 min bei 4°C
- im sauberen Laborraum bei Raumtemperatur (25°C) und mind. 60% Luftfeuchte leicht beschlagen (feucht aber nicht nass) lassen
- dann *UV-Crosslinking* mit 2 x 300 J/cm² zur irreversiblen Anbindung der Hybridmoleküle an die Chipoberfläche.

Anschließend wurden 30µl des Extensionsreaktionsgemischs mit der Zusammensetzung:

| | |
|---|---|
| 6 µl | 5x Sequenase-Puffer (Amersham) |
| 2,4 µl | 1µM Cy5-ddATP (NEN) |
| 2,4 µl | 1 µM R110-ddGTP (PerkinElmer) |
| 6,1 U | Sequenase™ |
| ad 30 µl | tridest. Wasser |

auf den *Microarray* aufgebracht. Die Extensionsreaktion erfolgte bei 50°C für 1h in der verdunstungssicheren Kammerkonstruktion des Thermocyclers *in situ* PCR System 1.000 (Perkin Elmer *in situ* PCR System 1.000 plus Zubehör) unter der zugehörigen Silikonmatte und Klammer (AmpliCover Clip und AmpliCover Disc, Fa. PerkinElmer).

Der Chip wurde dann 5 sec auf Eis gekühlt, die Klammer geöffnet und unmittelbar mit heißem dest. Wasser bei 85°C und mit 1x SSC mit 0,1% SDS bei 65°C (Sambrook et al., 2001, vide supra) gewaschen. Nach kurzem Spülen mit dest. Wasser wurde der Chip im Stickstoffstrom getrocknet.

Der Chip wurde mit dem Laserscanner GenTAC LS4 (Genomic Solutions, USA) analysiert. Dazu wurden die Probenpunkte mit den nun fluoreszenzmarkierten verlängerten Extensionsprimern mit monochromatischem Licht bei 500nm und 650nm angeregt und das emittierte Licht bei 525nm und 667nm gemessen und registriert. Die physikalischen Eigenschaften der Farbstoffe wurden bei der Wahl der Laser/Filter-Systeme und der eingesetzten Laserenergien nach Empfehlung des Geräteherstellers (Manual der Firma Genomic Solutions, USA) berücksichtigt.

Die Ergebnisse (Basenzustände in der jeweiligen SNP-Position) wurden als Intensitätswerte der Fluoreszenz direkt in einer Datenbank abgelegt. Die benutzte Software war GT Scan-Software, GSI Analyzer (sowie zugehörige Dokumentation).

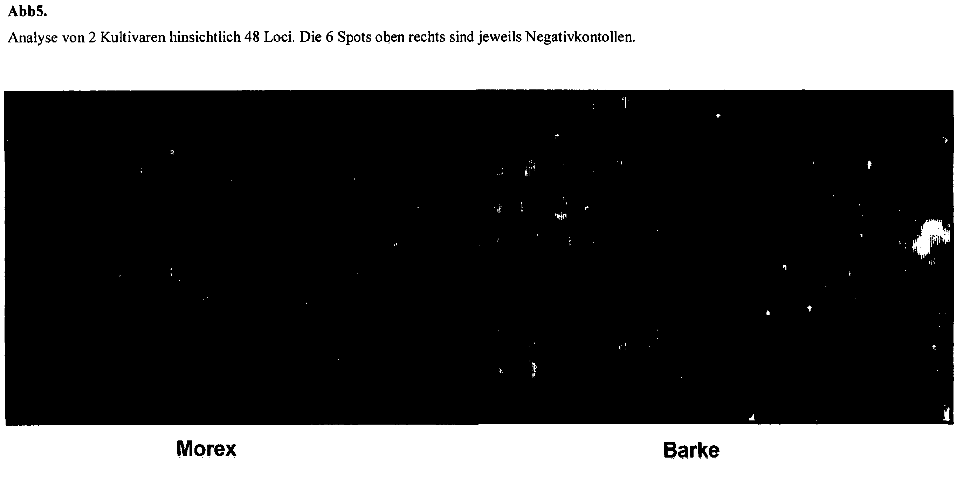

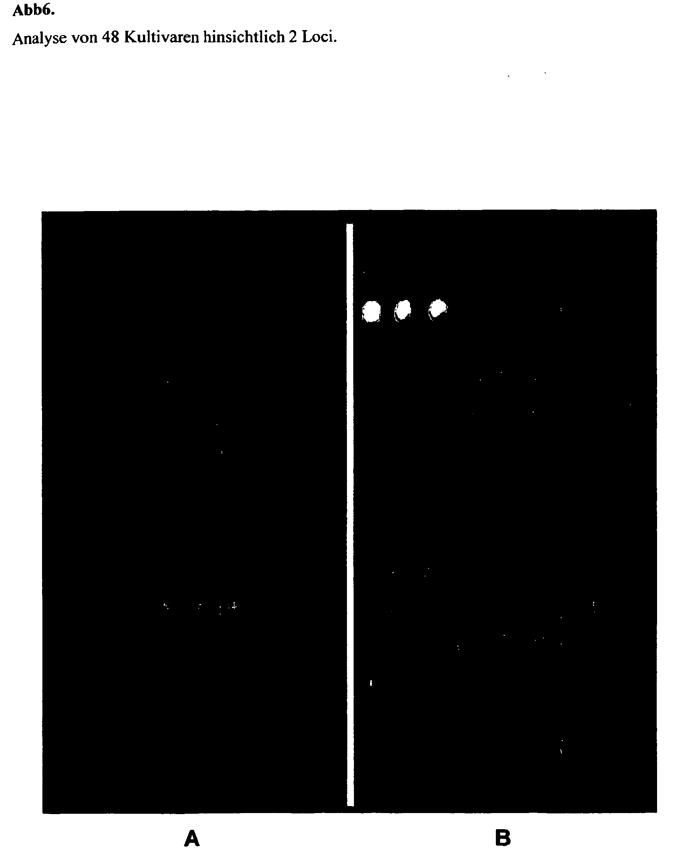

In Abb. 5 sind die Ergebnisse des Screenens von 48 Loci in den 2 Kultivaren Morex und Barke gezeigt. Jedes der 48 SNP Sondenmoleküle wurde wie beschrieben in Mikrotiterplatten mit den dazugehörigen einzelsträngigen PCR Fragmenten eines Gerstenindividuums (Morex oder Barke) gemischt und hybridisiert. Jedes der 48 Sonden-Hybridmoleküle wurde 3-fach redundant aufgespottet (jeweils 3 aufeinanderfolgende Punkte sind die selben Sondenhybride aus dem selben Well der Mikrotiterplatte) und dann die Extension auf dem Chip durchgeführt. Hierfür wurde, da es sich um C-T-Polymorphismen handelt, mit Cy5 markiertes ddTTP und mit R110 markiertes ddCTP verwendet. Die Darstellung von ddCTP ist eine Falschfarbe, da R110 blau fluoresziert, die Darstellung in grün ist jedoch angenehmer für das Auge.

Die Basenfolge an den einzelnen Loci in den Gersten-Individuen Barke und Morex kann einfach abgelesen werden (Rot = Adenin, Grün = Cytosin).

### Beispiel 2: Nachweis von 2 Loci in 48 Kultivaren

Im folgenden wird der Nachweis von 2 Loci A und B in 48 Kultivaren der Gerste beschrieben. Die Identifizierung der SNPs, die Herstellung der Probenmoleküle und Sonden, die Versuchsdurchführung und die Auswertung erfolgten analog zu Beispiel 1. Im Beispiel werden SNPs bezüglich Adenin (A) und Cytosin (C) untersucht.

Jeder der 2 SNP Loci wurde wie beschrieben mit den entsprechenden Primerpaaren aus 48 verschiedenen Kultivaren der Gerste amplifiziert (Morex, Barke und 46 weitere) und einzelsträngig gemacht. Die 48 Einzelstränge wurden mit dem Sondenmolekül des jeweiligen SNPs hybridisiert und wie unter Beispiel 1 dreifach redundant gespottet (jeweils 3 aufeinander-folgende Punkte stellen das selbe Kultivar dar, d.h. sie wurden in ein und dem selben Well der Mikrotiterplatte hybridisiert). Die Extension wurde auf dem Chip mit R110-ddCTP und Cy5-ddATP durchgeführt. Die Darstellung von ddCTP ist eine Falschfarbe, da R110 blau fluoresziert, die Darstellung in grün ist jedoch angenehmer für das Auge.

Hier kann direkt der Polymorphiegrad der Marker beurteilt werden. Die Basenabfolge an einem Locus (A oder B) kann wie bei einem Sequenzchromatogramm einfach abgelesen werden. Von links unten z.B. bei Locus A: Gersten 1-5: Cytosin, Gerste 6: Adenin, die nächsten 10 Gersten wieder Cytosin und die nächste Gerste ein Adenin usw.

## Patentansprüche

1. Verfahren zum parallelen Nachweis von Nukleinsäuren auf einem gemeinsamen Träger, umfassend die nachfolgenden Schritte in der angegebenen Reihenfolge:
a) Herstellen von Hybriden durch Mischen und Hybridisieren jeweils eines ersten einzelsträngigen Nukleinsäuremoleküls (Probenmoleküls) und eines zweiten einzelsträngigen Nukleinsäuremoleküls (Sondenmolekül), dessen Sequenz zu der des Probenmoleküls mindestens teilweise komplementär ist, so dass das Proben- und das Sondenmolekül mindestens über die komplementären Sequenzen miteinander hybridisieren können,
b) Anordnen der einzelnen Hybride aus Schritt a) auf einem gemeinsamen Träger in Form diskreter Bereiche und Immobilisieren der Hybride auf dem Träger über das Sondenmolekül, und
c) Nachweis der Hybridisierung mit einem geeigneten Nachweisverfahren.

2. Verfahren nach Anspruch 1 zum Nachweis eines Nukleotidpolymorphismus (SNP), wobei das Probenmolekül den nachzuweisenden SNP aufweist.

3. Verfahren nach Anspruch 2,
wobei Sondenmoleküle, die jeweils verschiedene Loci repräsentieren, und Probenmoleküle verschiedener Individuen zur parallelen Analyse verwendet werden.

4. Verfahren nach Anspruch 2 oder 3,
wobei in Schritt c) der Nachweis des SNP über Auswertung der Hybridisierungsreaktion durch enzymatische Template-abhängige Extension des zweiten Nukleinsäuremoleküls, das innerhalb des Hybrids als Primer fungiert, unter Einbau von markierten Nukleotiden oder - analoga erfolgt.

5. Verfahren nach Anspruch 4,
wobei der Nachweis durch enzymatische Template-abhängige Extension des zweiten Nukleinsäuremoleküls, das innerhalb des Hybrids als Primer fungiert, unter Einbau eines zum Kettenabbruch führenden markierten Nukleotids oder -analogons erfolgt.

6. Verfahren nach Anspruch 4 oder 5,
wobei es sich bei den Nukleotidanaloga um ddNTPs handelt und N für A, T, G, C steht.

7. Verfahren nach Anspruch 4 oder 5,
wobei die zur Markierung verwendeten Moleküle Cyanin-Farbstoffe, bevorzugt Cy3 und/oder Cy5, Renaissance-Farbstoffe, bevorzugt ROX und/oder R110 und/oder Fluorescein-Farbstoffe, bevorzugt FAM und/oder FITC umfassen.

8. Verfahren nach einem der Ansprüche 1 bis 7,
wobei die Sondenmoleküle eine Länge von mindestens 10 bis 100 Nukleotiden, bevorzugt von mindestens 20 bis 75 Nukleotiden, besonders bevorzugt von mindestens 20 bis 50 Nukleotiden, insbesondere bevorzugt von mindestens 20 bis 40 Nukleotiden, ebenfalls insbesondere bevorzugt von 20 bis 35 Nukleotiden und am meisten bevorzugt von mindestens 20 bis 30 Nukleotiden aufweisen.

9. Verfahren nach einem der Ansprüche 1 bis 8,
wobei die Probenmoleküle eine Länge von mindestens 30 bis 250 Nukleotiden, von mindestens 30 bis 200 Nukleotiden, bevorzugt von mindestens 40 bis 180 Nukleotiden, besonders bevorzugt von mindestens 50 bis 180 Nukleotiden, insbesondere bevorzugt von mindestens 70 bis 160 Nukleotiden und am meisten bevorzugt von etwa 80 bis 140 Nukleotiden aufweisen.

10. Verfahren nach einem der Ansprüche 1 bis 9,
wobei mindestens 1 bis 20000 unterschiedliche Sonden, mindestens 48 bis 15360 unterschiedliche Sonden, bevorzugt mindestens 96 bis 15360 unterschiedliche Sonden, besonders bevorzugt mindestens 96 bis 6144 unterschiedliche Sonden, insbesondere bevorzugt mindestens 96 bis 1536 unterschiedliche Sonden und am meisten bevorzugt mindestens 96 bis 384 unterschiedliche Sonden verwendet werden.

11. Verfahren nach einem der Ansprüche 1 bis 10,
wobei Probenmoleküle von mindestens 1 bis 20000 unterschiedlichen Individuen, mindestens 48 bis 15360 unterschiedlichen Individuen, bevorzugt mindestens 96 bis 15360 unterschiedlichen Individuen, besonders bevorzugt mindestens 96 bis 6144 unterschiedlichen Individuen, insbesondere bevorzugt mindestens 96 bis 1536 unterschiedlichen Individuen bezüglich mindestens 1 bis 20000 unterschiedlicher Loci, mindestens 48 bis 15360 unterschiedlicher Loci, bevorzugt mindestens 96 bis 15360 unterschiedlicher Loci, besonders bevorzugt mindestens 96 bis 6144 unterschiedlicher Loci, insbesondere bevorzugt mindestens 96 bis 1536 unterschiedlicher Loci und am meisten bevorzugt mindestens 96 bis 384 unterschiedlicher Loci parallel untersucht werden.

12. Verfahren nach einem der Ansprüche 1 bis 11,
wobei die einzelsträngigen Probenmoleküle durch PCR-Amplifikation und selektiven enzymatischen Verdau eines Strangs, durch asymmetrische PCR und/oder durch Affinitätsreinigung eines der beiden amplifizierten Stränge erhalten werden.

13. Verfahren nach einem der Ansprüche 1 bis 12,
wobei die einzelsträngigen Sondenmoleküle durch chemische Synthese erhalten werden.

## Claims

1. Method for parallel detection of nucleic acids on a common carrier comprising the following steps in the identified order:
a) Producing hybrids by mixing and hybridizing of a first single stranded nucleic acid molecule (sample molecule) and a second single stranded nucleic acid molecule (probe molecule), whose sequence is at least partially complementary to the sequence of the sample molecule, so that at least the complementary sequences of the sample molecule and the probe molecule are able to hybridize with each other,
b) arranging the individual hybrids from step a) on a common carrier in the form of discrete areas and immobilizing the hybrids on the carrier via the probe molecule, and
c) detecting the hybridization by means of a suitable detection method.

2. Method according to claim 1 for the detection of a single nucleotide polymorphism (SNP), wherein the sample molecule exhibits the SNP to be detected.

3. Method according to claim 2,
wherein probe molecules that represent different loci and sample molecules of different individuals are used for parallel analysis.

4. Method according to claim 2 or 3,
wherein in step c) the detection of the SNP occurs through analysis of the hybridization reaction by incorporation of labelled nucleotides or nucleotide analogues via enzymatic template-dependent extension of the second nucleic acid molecule that acts as a primer within the hybrid.

5. Method according to claim 4,
wherein the detection via enzymatic template-dependent extension of the second nucleic acid molecule that acts as a primer within the hybrid occurs by incorporation of a labelled nucleotide or nucleotide analogue that causes chain termination.

6. Method according to claim 4 or 5,
wherein the nucleotide analogues are ddNTPs and N stands for A, T, G, C.

7. Method according to claim 4 or 5,
wherein the molecules used for labelling comprise cyanine dyes, preferably Cy3 and/or Cy5, renaissance dyes, preferably ROX and/or R110 and/or fluorescein dyes, preferably FAM and/or FITC.

8. Method according to any of claims 1 to 7,
wherein the probe molecules have a length of at least 10 to 100 nucleotides, preferably of at least 20 to 75 nucleotides, more preferably of at least 20 to 50 nucleotides, more preferably of at least 20 to 40 nucleotides, also more preferably of at least 20 to 35 nucleotides and most preferably of at least 20 to 30 nucleotides.

9. Method according to any of claims 1 to 8,
wherein the sample molecules have a length of at least 30 to 250 nucleotides, of at least 30 to 200 nucleotides, preferably of at least 40 to 180 nucleotides, preferably of at least 50 to 180 nucleotides, preferably of at least 70 to 160 nucleotides and most preferably of about 80 to 140 nucleotides.

10. Method according to any of claims 1 to 9,
wherein at least 1 to 20000 different probes, at least 48 to 15360 different probes, preferably at least 96 to 15360 different probes, preferably at least 96 to 6144 different probes, more preferably at least 96 to 1536 different probes and most preferably at least 96 to 384 different probes are used.

11. Method according to any of claims 1 to 10,
wherein sample molecules of at least 1 to 20000 different individuals, of at least 48 to 15360 different individuals, preferably of at least 96 to 15360 different individuals, preferably of at least 96 to 6144 different individuals, more preferably of at least 96 to 1536 different individuals with respect to at least 1 to 20000 different loci, at least 48 to 15360 different loci, preferably at least 96 to 15360 different loci, preferably at least 96 to 6144 different loci, more preferably at least 96 to 1536 different loci and most preferably at least 96 to 384 different loci are analyzed in parallel.

12. Method according to any of claims 1 to 11,
wherein the single stranded sample molecules are obtained by PCR amplification and selective enzymatic digestion of a strand, by asymmetric PCR and/or by affinity purification of one of the two amplified strands.

13. Method according to any of claims 1 to 12,
wherein the single stranded probe molecules are obtained by chemical synthesis.

## Revendications

1. Procédé de détection parallèle d'acides nucléiques sur un support commun comprenant les étapes suivantes dans l'ordre indiqué :
a) Elaboration d'hybrides en procédant à un mélange et hybridation d'une première molécule d'acide nucléique (molécule échantillon) simple brin et d'une deuxième molécule d'acide nucléique (molécule-sonde) simple brin dont la séquence est du moins en partie complémentaire de celle de la molécule échantillon de sorte que la molécule échantillon et la molécule-sonde peuvent hybrider ensemble au moins par l'intermédiaire des séquences complémentaires,
b) agencement des différents hybrides obtenus dans l'étape a) sur un support commun sous forme de régions distinctes et immobilisation des hybrides sur le support par l'intermédiaire de la molécule-sonde, et
c) détection de l'hybridation moyennant un procédé de détection approprié.

2. Procédé selon la revendication 1 de détection d'un polymorphisme nucléotidique (SNP), dans lequel la molécule échantillon comporte le SNP qu'il y a lieu de détecter.

3. Procédé selon la revendication 2,
dans lequel on utilise des molécules-sonde représentant chacune un autre locus et des molécules échantillon de différents individus pour faire une analyse parallèle.

4. Procédé selon la revendication 2 ou 3,
dans lequel, dans l'étape c), la détection du SNP se fait par évaluation de la réaction d'hybridation par extension enzymatique, dépendante de la matrice, de la deuxième molécule d'acide nucléique faisant office d'amorce dans l'hybride, via incorporation de nucléotides ou d'analogues de nucléotides marqués.

5. Procédé selon la revendication 4,
dans lequel la détection se fait par extension enzymatique, dépendante de la matrice, de la deuxième molécule d'acide nucléique faisant office d'amorce dans l'hybride, via incorporation d'un nucléotide ou d'un analogue de nucléotide marqués provoquant la terminaison de la chaîne.

6. Procédé selon la revendication 4 ou 5,
dans lequel les analogues de nucléotides sont des ddNTP et N est mis pour A, T, G, C.

7. Procédé selon la revendication 4 ou 5,
dans lequel les molécules utilisées pour le marquage comprennent des colorants à base de cyanine, de préférence Cy3 et/ou Cy5, des colorants Renaissance, de préférence ROX et/ou R110, et/ou des colorants à base de fluorescéine, de préférence FAM et/ou FITC.

8. Procédé selon l'une quelconque des revendications 1 à 7,
dans lequel les molécules-sonde ont une longueur comprise entre au moins 10 et 100 nucléotides, de préférence entre au moins 20 et 75 nucléotides, la préférence étant accordée à une longueur comprise entre au moins 20 et 50 nucléotides, une préférence particulière étant accordée à une longueur comprise entre au moins 20 et 40 nucléotides, une longueur comprise entre 20 et 35 nucléotides étant également préférée, la préférence la plus prononcée étant accordée à une longueur comprise entre 20 et 30 nucléotides.

9. Procédé selon l'une quelconque des revendications 1 à 8,
dans lequel les molécules échantillon ont une longueur comprise entre au moins 30 et 250 nucléotides, entre au moins 30 et 200 nucléotides, de préférence entre au moins 40 et 180 nucléotides, la préférence étant accordée à une longueur comprise entre au moins 50 et 180 nucléotides, une préférence particulière étant accordée à une longueur comprise entre au moins 70 et 160 nucléotides, la préférence la plus prononcée étant accordée à une longueur comprise entre environ 80 et 140 nucléotides.

10. Procédé selon l'une quelconque des revendications 1 à 9,
dans lequel on utilise entre au moins 1 et 20000 sondes différentes, entre au moins 48 et 15360 sondes différentes, de préférence entre au moins 96 et 15360 sondes différentes, la préférence étant accordée à un nombre de sondes différentes compris entre au moins 96 et 6144, une préférence particulière étant accordée à un nombre de sondes différentes compris entre au moins 96 et 1536, la préférence la plus prononcée étant accordée à un nombre de sondes différentes compris entre au moins 96 et 384.

11. Procédé selon l'une quelconque des revendications 1 à 10,
dans lequel on analyse parallèlement des molécules échantillon d'entre au moins 1 et 20000 individus différents, d'entre au moins 48 et 15360 individus différents, de préférence entre au moins 96 et 15360 individus différents, la préférence étant accordée à un nombre de d'individus différents compris entre au moins 96 et 6144, une préférence particulière étant accordée à un nombre d'individus différents compris entre au moins 96 et 1536, en référence à entre au moins 1 et 20000 locus différents, à entre au moins 48 et 15360 locus différents, de préférence entre au moins 96 et 15360 locus différents, la préférence étant accordée à un nombre de locus différents compris entre au moins 96 et 6144, une préférence particulière étant accordée à un nombre de locus différents compris entre au moins 96 et 1536, la préférence la plus prononcée étant accordée à un nombre de locus différents compris entre au moins 96 et 384.

12. Procédé selon l'une quelconque des revendications 1 à 11,
dans lequel les molécules échantillon simple brin sont obtenues par amplification PCR et digestion enzymatique sélective d'un brin, par PCR asymétrique et/ou par purification par affinité d'un des deux brins amplifiés.

13. Procédé selon l'une quelconque des revendications 1 à 12,
dans lequel les molécules-sonde simple brin sont obtenues par synthèse chimique.


### SEQUENZPROTOKOLL

<110> IPK Institut für Pflanzengenetik und Kulturpflanze
<120> Verfahren zum Nachweis von SNPs auf polydimensionalen Microarrays
<130> 17408
<140> PCT/EP 03/10773
   <141> 2003-09-26
<150> DE 102 45 145.1
   <151> 2002-09-27
<160> 13
<170> PatentIn Ver. 2.1
<210> 1
   <211> 117
   <212> DNA
   <213> Hordeum vulgare
<400> 1
   ggtggcaaaa agtctagcag tagtagttcc ctgatgtacg aagctcccct cggctacagc 60
   attgaagacg ttcgaccagc tggaggcgca aagaagtttt ctgctgcata ytcaaac    117
<210> 2
   <211> 109
   <212> DNA
   <213> Hordeum vulgare
<400> 2
   tgcgcgaaga agccatcctg atatcgtttt tggctttccc cttcccgtag tttaggattt 60
   ttaactgctg caattttatt ctgactcttt tctcccacca atctctctg 109
<210> 3
   <211> 23
   <212> DNA
   <213> Künstliche Sequenz
<220>
   <223> Beschreibung der künstlichen Sequenz: Oligonucleotid
<400> 3
   agaagttttc tgctgcatay tca 23
<210> 4
   <211> 24
   <212> DNA
   <213> Künstliche Sequenz
<220>
   <223> Beschreibung der künstlichen Sequenz: Oligonucleotid
<400> 4
   tggctttccc cttcccgtag ttta 24
<210> 5
   <211> 21
   <212> DNA
   <213> Künstliche Sequenz
<220>
   <223> Beschreibung der künstlichen Sequenz: Oligonucleotid
<400> 5
   taaactacgg gaaggggaaa g 21
<210> 6
   <211> 19
   <212> DNA
   <213> Künstliche Sequenz
<220>
   <223> Beschreibung der künstlichen Sequenz: Oligonucleotid
<400> 6
   agaagttttc tgctgcata 19
<210> 7
   <211> 24
   <212> DNA
   <213> Künstliche Sequenz
<220>
   <223> Beschreibung der künstlichen Sequenz: Oligonucleotid
<400> 7
   taaactacgg gaaggggaaa gcca 24
<210> 8
   <211> 23
   <212> DNA
   <213> Künstliche Sequenz
<220>
   <223> Beschreibung der künstlichen Sequenz: Oligonucleotid
<400> 8
   tgaytatgca gcagaaaact tct 23
<210> 9
   <211> 23
   <212> DNA
   <213> Künstliche Sequenz
<220>
   <223> Beschreibung der künstlichen Sequenz: Oligonucleotid
<400> 9
   tcttcaaaag acgacgtaty agt 23
<210> 10
   <211> 24
   <212> DNA
   <213> Künstliche Sequenz
<220>
   <223> Beschreibung der künstlichen Sequenz: Oligonucleotid
<400> 10
   accgaaaggg gaagggcatc aaat 24
<210> 11
   <211> 19
   <212> DNA
   <213> Künstliche Sequenz
<220>
   <223> Beschreibung der künstlichen Sequenz: Oligonucleotid
<400> 11
   atacgtcgtc ttttgaaga 19
<210> 12
   <211> 23
   <212> DNA
   <213> Künstliche Sequenz
<220>
   <223> Beschreibung der künstlichen Sequenz: Oligonucleotid
<400> 12
   tcttcaaaag acgacgtaty tca 23
<210> 13
   <211> 20
   <212> DNA
   <213> Künstliche Sequenz
<220>
   <223> Beschreibung der künstlichen Sequenz: Oligonucleotid
<400> 13
   ratacgtcgt cttttgaaga 20